# EUROPÄISCHE PATENTSCHRIFT

(11) **EP 3 903 747 B1**
(45) Veröffentlichungstag und Bekanntmachung des Hinweises auf die Patenterteilung: **23.09.2026**
(21) Anmeldenummer: 21175706.7
(22) Anmeldetag: 24.01.2019
(51) Int. Cl.: A61F 5/41

(54) **STIMULATIONSVORRICHTUNG FÜR EINEN MÄNNLICHEN PENIS**
STIMULATION DEVICE FOR A MALE PENIS
DISPOSITIF DE STIMULATION POUR UN PÉNIS HUMAIN

(43) Veröffentlichungstag der Anmeldung: 03.11.2021
(62) Dokumentnummer(n) der früheren Anmeldung(en) nach Art. 76 EPÜ: 19153494.0 / 3 685 809
(73) Patentinhaber: Novoluto GmbH, 10249 Berlin (DE)
(72) Erfinder: Kirsten, Enrico, 10435 Berlin (DE); Zegenhagen, Mark Tobias, 10115 Berlin (DE)
(74) Vertreter: Araujo Edo, Mario

(56) Entgegenhaltungen:
- DE-U1- 202015 005 041
- US-A- 5 647 837
- US-A1- 2015 105 609
- US-A1- 2016 213 557

## Beschreibung

Die Erfindung betrifft eine Stimulationsvorrichtung für einen männlichen Penis und insbesondere einen Applikator für eine solche Stimulationsvorrichtung. Die Stimulationsvorrichtung weist eine Druckfelderzeugungseinrichtung zur Erzeugung eines pneumatischen Druckwechselfeldes auf, welches mittels des Applikators auf einen zu stimulierenden Bereich eines Penis appliziert werden kann.

Es sind verschiedene Stimulationsvorrichtungen für den männlichen Penis bekannt, welche zu einer sexuellen Erregung führen beziehungsweise eine sexuelle Erregung gegebenenfalls bis zum Höhepunkt steigern können. Derartige Stimulationsvorrichtungen weisen üblicherweise einen Aufnahmeraum auf, welcher im Wesentlichen geschlossen ist und eine Öffnung aufweist, in welche der (erigierte) Penis eingeführt werden kann. Oftmals ist das vordere Ende des Aufnahmeraums verschlossen, sodass insbesondere die Eichel des Penis umschlossen ist. Die Innenseite des Aufnahmeraums liegt am Penis an und kann zur Verstärkung der Stimulation Strukturen, wie beispielsweise Noppen aufweisen. Neben manuellen Vorrichtungen, bei denen ein Benutzer selbst gewünschte Bewegungen zur Stimulation durch Reibung ausführen muss, sind automatische Vorrichtungen bekannt.

Derartige automatische Vorrichtungen können entweder eine manuelle Bewegung nachahmen oder andere stimulationsartige Bewegungen ausführen. Beispielsweise kann der Aufnahmeraum ganz oder teilweise in seiner Form oder seinem Volumen durch auf den Aufnahmeraum wirkende mechanische oder pneumatische Vorrichtungen verändert werden, um eine Stimulation zu erzeugen. Der Aufnahmeraum kann dazu als flexible Hülle aus einem weichen Material gestaltet sein und sich in einem festen Gehäuse befinden, welches neben dem Aufnahmeraum entsprechende Antriebe, Pumpen, Stimulationsköpfe, Batterien und dergleichen beherbergen kann. Bekannt sind auch Stimulationsvorrichtungen, bei denen der Aufnahmeraum am Schaft des Penis abgedichtet wird, sodass mittels eines variierenden Unterdrucks eine Stimulationswirkung erzielt werden kann.

Da bei den bekannten Vorrichtungen oftmals lediglich eine Stimulationsfunktion realisiert ist, wird der Stimulationseffekt oftmals als nicht zufriedenstellend wahrgenommen. Außerdem wird bei bekannten Stimulationsvorrichtungen zumeist der gesamte Penis in den Aufnahmeraum eingeführt, sodass auch aufgrund des großen Gehäuses diese Vorrichtungen relativ unhandlich sind. Auch sind solche Vorrichtungen meist nur in einer Standardgröße erhältlich und sind für verschiedene Penisgrößen oft unzureichend anpassungsfähig. Durch die geschlossene Form des Aufnahmeraums kann zudem die Reinigung schwierig sein, auch da oftmals Gleitgel oder ähnliches notwendig ist, um Hautreizungen durch Reibung zu vermeiden.

Im Dokument DE 20 2015 005 041 U1 wird eine manuelle Stimulationsvorrichtung beschrieben, welche einen äußeren Becherkörper mit einer innenliegenden Gummihülse aufweist. Der Penis wird durch eine Öffnung auf einer Seite in die Gummihülse eingeführt, während der Becherkörper am anderen Ende eine Dichtgruppe aufweist, welche derart einstellbar ist, dass sich bei einer Bewegung der Vorrichtung die Stärke der Unter- bzw. Überdrücke einstellen lässt, die durch die Bewegung entstehen. Eine zusätzliche Pumpe ist nicht vorgesehen.

Es sind auch Vorrichtungen bekannt, mit denen ein Unterdruck auf einen Penis aufgebracht werden kann. Beispielsweise ist aus dem Dokument US 5,647,837 eine Vorrichtung zum Aufbau einer Erektion bekannt. Der Penis wird in einen zylindrischen Hohlkörper durch eine Öffnung mit einer umlaufenden Dichtung eingeführt. Es ist eine Pumpe angeschlossen, mit der dann ein Unterdruck erzeugt werden kann. Aus dem Dokument US 2015/0105609 A1 ist eine Vorrichtung bekannt, in welche der Penis eingeführt wird und dann ein Unterdruck zur Penisverlängerung erzeugt werden soll.

Aus der US 2016/0213557 A1 ist eine Vorrichtung zur druckwechselfeldbasierten Stimulation einer Klitoris bekannt, die keinen Aufnahmeraum zum Aufnehmen eines Penis aufweist, in den eine Öffnung eines Druckraums direkt mündet und der zwei entlang einer Längsrichtung gegenüberliegende offene Enden aufweist.

Aufgabe der vorliegenden Erfindung ist es daher, eine Stimulationsvorrichtung für einen männlichen Penis zu schaffen, welche hinsichtlich Stimulation des Penis und Anwendung verbessert ist.

Die Aufgabe wird gelöst durch einen Applikator für eine Stimulationsvorrichtung für einen männlichen Penis und eine Stimulationsvorrichtung mit einem solchen Applikator mit den Merkmalen der unabhängigen Ansprüche. Es ist ferner ein Artikel mit einer derartigen Stimulationsvorrichtung und mindestens einem weiteren Applikator vorgesehen.

Gemäß einem ersten Aspekt ist ein Applikator für eine Stimulationsvorrichtung für einen männlichen Penis geschaffen. Der Applikator umfasst einen Applikatorkörper mit einem Anlagebereich, welcher eingerichtet ist, beim Anlegen an einen Penis hieran wenigstens teilweise zur Anlage zu kommen. Des Weiteren umfasst der Applikator einen Druckraum, welcher an dem Applikatorkörper gebildet und eingerichtet ist, von einer Druckfelderzeugungseinrichtung ein pneumatisches Druckwechselfeld zu empfangen. Der Druckraum weist eine Öffnung im Anlagebereich auf, sodass das Druckwechselfeld über die Öffnung auf einen zu stimulierenden Bereich des Penis applizierbar ist. Eine Dichteinrichtung ist an dem Applikatorkörper gebildet und eingerichtet, beim Anlegen des Applikatorkörpers an den Penis den Druckraum gegenüber der Umgebung abzudichten.

Gemäß einem weiteren Aspekt ist eine Stimulationsvorrichtung für einen männlichen Penis geschaffen, welche zumindest einen derartigen Applikator und eine Druckfelderzeugungseinrichtung zur Erzeugung eines pneumatischen Druckwechselfeldes umfasst. Die Druckfelderzeugungseinrichtung weist einen pneumatischen Ausgang auf, über welchen ein erzeugtes pneumatisches Druckwechselfeld ausgebbar ist, wobei der pneumatische Ausgang mit dem Applikator verbindbar oder verbunden ist, sodass das pneumatische Druckwechselfeld auf den Druckraum des Applikators übertragbar ist.

Gemäß noch einem weiteren Aspekt ist ein Artikel mit einer derartigen Stimulationsvorrichtung für einen männlichen Penis und wenigstens einem weiteren derartigen Applikator geschaffen, wobei die in dem Artikel enthaltenen Applikatoren vorzugsweise verschieden sind. Der Artikel kann auch als Set bezeichnet werden und erlaubt einen modularen Aufbau der Stimulationsvorrichtung.

Gemäß einem bevorzugten Ausführungsbeispiel ist der Applikator lösbar mit der Druckfelderzeugungseinrichtung verbunden, entweder direkt oder mittels eines Verbindungsteils wie unten detaillierter beschrieben, sodass er zur Reinigung oder zum Austausch von der Druckfelderzeugungseinrichtung getrennt und wieder mit dieser verbunden werden kann. Alternativ kann der Applikator jedoch fest mit der Druckfelderzeugungseinrichtung verbunden beziehungsweise integral mit dieser gebildet sein.

Applikatoren eines Artikels beziehungsweise Sets können insbesondere hinsichtlich Form, Größe, Geometrie und dergleichen verschieden sein. Auch können sich die Applikatoren in weiteren zuvor und im Folgenden beschriebenen funktionalen Teilen, wie beispielsweise der Dichteinrichtung oder der Gestalt des Anlagebereichs unterscheiden. Es kann vorgesehen sein, dass sich die Applikatoren hinsichtlich der Geometrie und/oder Größe ihres Druckraums unterscheiden, wodurch bei gleichbleibendem Betrieb der Druckfelderzeugungseinrichtung Stimulationen unterschiedlicher Intensität bewirkt werden können. Alternativ kann jedoch vorgesehen sein, dass die Applikatoren gleich sind, sodass ein Applikator bei Verlust, Beschädigung, Abnutzung oder dergleichen ersetzt werden kann. Besonders vorteilhaft sind die Applikatoren dazu lösbar mit der Druckfelderzeugungseinrichtung verbindbar, beispielsweise über ein Verbindungsteil wie unten detaillierter beschrieben, welches ebenfalls als Teil des Artikels angesehen werden kann. Der Artikel kann in einer Variante mehr als ein Verbindungsteil umfassen, wobei die Verbindungsteile gleich oder verschieden sein können.

Mittels des Applikators kann ein von der Druckfelderzeugungseinrichtung erzeugtes Druckwechselfeld auf einen zu stimulierenden Teil des Penis appliziert werden. Dazu wird der Applikator mit dem Anlagebereich auf einen zu stimulierenden Teil des Penis, beispielsweise die Eichel, die Eichelkranzfurche und oder das Frenulum (Vorhautbändchen) aufgelegt, wie weiter unten detaillierter beschrieben wird. Die Öffnung des Druckraums ist im Anlagebereich angeordnet und weist bei Benutzung in Richtung des Penis, sodass im Bereich der Öffnung des Druckraums kein Kontakt zwischen dem Penis und dem Applikator besteht und der Druckraum direkt an die Haut des Penis grenzt. Das Druckwechselfeld wird somit direkt auf den Penis appliziert.

Im Gegensatz zu bekannten Stimulationsvorrichtung erfolgt die stimulierende Anregung somit durch kontaktloses Übertragen einer Stimulationskraft mittel des Druckwechselfeldes. Insbesondere kommen auch Teile der Druckfelderzeugungseinrichtung, beispielsweise eine flexible Wand oder Membran der Druckfelderzeugungseinrichtung, die zur Druckwechselfelderzeugung entsprechend ausgelenkt wird, nicht in Kontakt mit dem Penis, insbesondere in keiner Phase des Betriebs. Mit anderen Worten, die flexible Wand oder flexible Membran ist zur Öffnung des Druckraums beabstandet und somit auch zur Dichteinrichtung beabstandet, insbesondere von dieser verschieden. Dies ist insbesondere für die Stimulation des empfindlichen Frenulums vorteilhaft, wie unten genauer beschrieben werden wird. Es kann somit ein besonderer Stimulationseffekt geschaffen werden, der sich vom Effekt üblicher taktiler Stimulationsvorrichtungen unterscheidet. Der Applikator ist kompakt und kann einfach an den Penis angelegt werden, sodass er einfach benutzt und auch einfach gereinigt werden kann.

Die Dichteinrichtung ist derart ausgestaltet, dass beim Anlegen des Applikators an den Penis der Druckraum gegenüber der Umgebung abgedichtet wird. Auf diese Weise kann sich ein Druckwechselfeld aufbauen und der zu stimulierende Teil des Penis, also insbesondere der Teil des Penis, welcher sich im Bereich der Öffnung des Druckraums unmittelbar angrenzend an den Druckraum befindet, mittels des Druckwechselfeldes stimuliert werden.

Unter Druckwechselfeld wird dabei ein solches variierendes Druckfeld verstanden, welches bezüglich des Umgebungsdrucks sowohl Unterdrücke als auch Überdrücke aufweist, beispielsweise abwechselnde Unterdruckphasen und Überdruckphasen oder in einem anderen vorgegebenen Muster von gegebenenfalls gleichen oder unterschiedlichen Unter- und Überdrücken. Dieses Druckwechselfeld herrscht im Druckraum des Applikators, insbesondere im Bereich der Öffnung des Druckraums, d.h. Parameter, wie Frequenz und Amplitude des Druckwechselfelds sind an der Öffnung zu messen. Der Druck kann dabei mit einer Frequenz von 5 Hz bis 250 Hz wechseln, vorzugsweise 10 Hz bis 200 Hz, weiter vorzugsweise 20 Hz bis 100 Hz, beispielsweise 60 Hz. Die Druckdifferenz kann 20 mbar bis 800 mbar betragen, vorzugsweise 50 mbar bis 750 mbar, weiter vorzugsweise 100 mbar bis 700 mbar, beispielsweise 200 mbar, wobei die Druckdifferenz zwischen dem höchsten Überdruck und dem niedrigsten Unterdruck besteht und vorzugsweise symmetrisch um einen Umgebungsdruck angeordnet ist. Bei einem Normaldruck der Umgebung von etwa 1 bar kann das Druckwechselfeld beispielsweise zu einem Druck von 0,7 bar bis 1,3 bar in dem Druckraum führen, was einer Druckdifferenz von 0,6 bar (600 mbar) entspricht. Das Druckwechselfeld ist insbesondere ein pneumatisches Druckwechselfeld, d. h. als Druckmedium wird insbesondere Luft verwendet. Es ist jedoch denkbar, dass das Druckwechselfeld über ein anderes Medium übertragen wird, beispielsweise über ein Fluid, wie Wasser, Gel oder dergleichen, welches in den Druckraum eingebracht wird.

Die Dichteinrichtung ist somit insbesondere dazu eingerichtet, den Druckraum sowohl bezüglich Unterdrücken als auch Überdrücken relativ zu einem Umgebungsdruck der Umgebung gegen die Umgebung abzudichten. Mit anderen Worten, die Dichteinrichtung ist eingerichtet, Strömungen, insbesondere Luftströmungen, in beide Richtungen, d.h. aus dem Druckraum in die Umgebung und von der Umgebung in den Druckraum zu verhindern oder zumindest annähernd zu verhindern. Zur Umgebung gehören insbesondere Bereiche außerhalb des Druckraums oder außerhalb des Applikators.

Die Dichteinrichtung kann insbesondere im Anlagebereich gebildet und eingerichtet sein, beim Anlegen des Applikatorkörpers zumindest abschnittsweise am Penis zur Anlage zu kommen, um den Druckraum gegenüber der Umgebung abzudichten. Insbesondere kann die Dichteinrichtung um die Öffnung des Druckraums herum umlaufend gebildet sein, beispielsweise kreisförmig, oval oder dergleichen. Die Dichteinrichtung kann dabei auch derart ausgebildet sein, dass sie mehrere Abschnitte umfasst, welche sich beim Aufbringen eines Anpressdrucks elastisch verformen, um eine umlaufende Dichtung zur Abdichtung des Druckraums gegenüber der Umgebung zu bilden.

Es versteht sich, dass die Dichteinrichtung auch andere Formen aufweisen kann, die zur dichtenden Anlage am Penis geeignet sind. Insbesondere ist jedoch vorzugsweise vorgesehen, dass die Dichteinrichtung den Penis nicht umlaufend umschließt, sondern seitlich am Penis anliegt, beispielsweise im Bereich des Frenulums. Die Dichteinrichtung kann alternativ oder zusätzlich auch zumindest abschnittweise in einem äußeren Randbereich des Applikatorkörpers gebildet sein, insbesondere im Anlagebereich.

Die Dichteinrichtung kann als erhabene Struktur im Anlagebereich ausgebildet sein und kann beispielsweise eine Dichtlippe, eine Dichtmembran, einen Wulst, einen Vorsprung oder dergleichen aufweisen. Vorteilhaft ist die Dichteinrichtung aus einem weichen Material gebildet, sodass sie sich beim Anlegen an den Penis den anatomischen Gegebenheiten anpasst und dichtend an der Haut des Penis anliegt. Die Dichteinrichtung kann zum Beispiel ein rundes, beispielsweise halbkreisförmiges Querschnittsprofil aufweisen. Es kann ein Anpressdruck auf die Dichteinrichtung aufgebracht werden, um die Abdichtung zu verbessern. Dies kann manuell oder durch eine Halteeinrichtung erfolgen, wie unten detaillierter beschrieben wird.

Die Dichteinrichtung kann insbesondere dazu ausgebildet sein, den Druckraum abzudichten, wenn der Applikator stationär am Penis anliegt, d. h. nicht bewegt wird. Die Dichteinrichtung kann jedoch derart beschaffen und als Gleitdichtung ausgebildet sein, dass zumindest eine teilweise Abdichtung des Druckraums oder auch eine annähernde Dichtung wie im unbewegten Zustand auch bei einer Bewegung des Applikators entlang des Penis erreicht wird. Vorteilhaft ist die Dichteinrichtung derart ausgebildet, dass sie trotz effektiver Abdichtung im unbewegten Zustand eine Bewegung des Applikators entlang des Penis zulässt oder zumindest nicht behindert. Beispielsweise kann die Dichteinrichtung dazu besonders weich und abgerundet ausgebildet sein. Gegebenenfalls kann bei einer Bewegung des Applikators durch die Dichteinrichtung eine zusätzliche Stimulation des Penis erreicht werden, wenn die Dichteinrichtung als erhabene Struktur im Anlagebereich ausgebildet ist.

Im Anlagebereich des Applikatorkörpers kann zumindest ein anatomisch geformter Vorsprung angeordnet sein, welcher vorzugsweise zumindest abschnittsweise dem Verlauf einer Eichelkranzfurche des Penis folgt. Der anatomisch geformte Vorsprung kann mit der Dichteinrichtung verbunden oder von dieser getrennt sein. Er kann die Dichtwirkung unterstützen und auch dazu dienen, eine korrekte Positionierung des Applikators am Penis zu erleichtern. Der anatomisch geformte Vorsprung kann hinsichtlich seiner Lage, seines Verlaufs und/oder seines Querschnittprofils an die gewünschte Anatomie angepasst sein. Beispielsweise kann der Vorsprung zumindest einem Teil der Eichelkranzfurche, beispielsweise im Bereich des Frenulums folgen. In diesem Fall kann der anatomisch geformte Vorsprung aus zwei Abschnitten gebildet sein, die einen Winkel einschließen. Der Vorsprung kann wie die Dichteinrichtung beispielsweise als Wulst oder Lippe ausgebildet sein.

Der Druckraum, welcher auch als Applikationsraum bezeichnet werden kann, ist vorzugsweise dazu eingerichtet, beim Anlegen des Applikatorkörpers an den Penis ein Frenulum des Penis zumindest teilweise aufzunehmen. Insbesondere ist die Öffnung des Druckraums im Anlagebereich des Applikatorkörpers dazu entsprechend bemessen und geformt, dass sie das Frenulum zumindest teilweise oder vollständig umgibt. Beispielsweise kann die Öffnung für die gesamte oder teilweise Aufnahme des Frenulums einen Durchmesser kleiner als 40 mm, vorzugsweise kleiner als 30 mm, weiter vorzugsweise kleiner als 20 mm aufweisen.

Da der Druckraum zum Penis hin offen ist, kann das Druckwechselfeld somit auf diese Weise direkt auf das Frenulum einwirken, beispielsweise durch leichtes Hineinziehen des Frenulums in den Druckraum in den Unterdruckphasen. Die Art der Stimulation durch ein pneumatisches Druckwechselfeld ist besonders effektiv, da die erogene Zone in der Unterdruckphase durch eine verstärkte Durchblutung sensibilisiert und in der Überdruckphase stimuliert wird. Diese Region des Penis zählt zu den stärksten erogenen Zonen des männlichen Körpers, sodass eine geeignete Stimulation zu sexueller Erregung führen beziehungsweise sexuelle Erregung steigern kann. Insbesondere die Stimulation mittels eines Druckwechselfeldes, also das kontaktlose Aufbringen einer Stimulationskraft, ist für das sensible Frenulum besonders geeignet.

Der Applikator kann einen Anschluss aufweisen, welcher an dem Applikatorkörper angeordnet und eingerichtet ist, an eine Druckfelderzeugungseinrichtung angeschlossen zu werden und von dieser ein pneumatisches Druckwechselfeld zu empfangen, wobei der Druckraum mit dem Anschluss fluidisch verbunden ist. Insbesondere ist es vorteilhaft, einen Anschluss vorzusehen, wenn der Applikator lösbar mit einer Druckfelderzeugungseinrichtung verbindbar ist. Insbesondere kann die Druckfelderzeugungseinrichtung auf einer Seite des Anschlusses und der Druckraum auf einer anderen, insbesondere gegenüberliegenden Seite des Anschlusses angeordnet sein. Beispielsweise eine zur Druckwechselfelderzeugung eingerichtete flexible Membran bildet somit keine Begrenzung des Druckraums, sondern ist außerhalb des Druckraums, vorzugsweise vom Druckraum beabstandet angeordnet.

In dem Applikatorkörper kann ein Fluidkanal gebildet sein, über welchen der Anschluss und der Druckraum fluidisch miteinander verbunden sind. Insbesondere kann der Fluidkanal einen kleineren Querschnitt aufweisen als der Druckraum beziehungsweise der Druckraum einen größeren Querschnitt als der Fluidkanal. Auf diese Weise kann das Druckwechselfeld effektiv vom Anschluss in den Druckraum übertragen werden.

Die Öffnung des Druckraums kann beispielsweise den gleichen Querschnitt wie der Druckraum aufweisen, sodass das Druckwechselfeld auf einer möglichst großen Fläche auf den zu stimulierenden Teil des Penis appliziert werden kann. Gleichzeitig kann der Anschluss an die Druckerzeugungseinrichtung im Querschnitt klein gehalten werden.

Um die Fläche, auf welche das Druckwechselfeld auf den Penis einwirkt, also insbesondere die Fläche der Öffnung des Druckraums im Anlagebereich weiter zu vergrößern, kann sich der Druckraum zur Öffnung hin erweitern. Alternativ kann der Druckraum jedoch einen gleich bleibenden Querschnitt aufweisen und beispielsweise zylindrisch ausgebildet sein.

Umgekehrt kann vorgesehen sein, dass sich der Querschnitt des Druckraums zur Öffnung hin verkleinert. Beispielsweise kann unabhängig davon auch ein Querschnitt des Fluidkanals und/oder Anschlusses größer sein als ein Querschnitt des Druckraums und/oder der Öffnung. Auf diese Weise kann der Druck im Bereich der Öffnung verstärkt werden. Auch kann unabhängig von der Geometrie des Druckraums die Öffnung eine kleinere Querschnittsfläche aufweisen als der an die Öffnung angrenzende Bereich des Druckraums.

Eine anschlussseitige Öffnung des Druckraums, über die das pneumatische Druckwechselfeld in den Druckraum einleitbar ist, kann der Öffnung des Druckraums im Anlagebereich des Applikatorkörpers gegenüberliegend angeordnet sein. Diese Anordnung ist vorteilhaft für die Übertragung des Druckwechselfeldes von der Druckerzeugungseinrichtung über den Anschluss in den Druckraum bis hin zur Öffnung des Druckraums und schließlich auf den Penis, da die Druckkräfte entlang einer Achse wirken. Alternativ kann es jedoch vorgesehen sein, dass der Anschluss an einer anderen Stelle im Applikator relativ zum Druckraum angeordnet ist, beispielsweise seitlich im Druckraum. Falls wie oben erwähnt ein Fluidkanal vorgesehen ist, kann der Fluidkanal entweder geradlinig verlaufen oder einen beliebigen anderen gekrümmten oder abgewinkelten Verlauf einnehmen, um den Anschluss mit dem Druckraum fluidisch zu verbinden.

Erfindungsgemäß definiert der Anlagebereich des Applikatorkörpers einen Aufnahmeraum, welcher durch den Anlagebereich begrenzt und eingerichtet ist, den Penis zumindest teilweise aufzunehmen, sodass der Anlagebereich entlang zumindest eines Teils des Umfangs des Penis anliegt. Da die Öffnung des Druckraums im Anlagebereich angeordnet ist, mündet sie direkt in den Aufnahmeraum. Mit anderen Worten, wie oben bereits beschrieben, ist der Druckraum zum Aufnahmeraum hin und damit auch zum Penis hin offen, wenn der Applikator am Penis anliegt, sodass das Druckwechselfeld direkt auf die Haut des Penis, beispielsweise das Frenulum, einwirken kann. Die Öffnung des Druckraums und die Dichteinrichtung können somit insbesondere in einer durch den Anlagebereich definierten Anlagefläche liegen.

Der Aufnahmeraum weist erfindungsgemäß zwei entlang einer Längsrichtung gegenüberliegende offene Enden auf, wobei der Penis durch eines der Enden in den Applikator eingeführt werden und gegebenenfalls aus dem anderen Ende wieder austreten kann. Hierzu kann der Aufnahmeraum beispielsweise symmetrisch bezüglich einer Ebene senkrecht zur Längsachse ausgebildet sein. Der Aufnahmeraum kann entlang seines Umfangs geschlossen oder offen sein. Wenn der Aufnahmeraum entlang des Umfangs offen ist, liegt der Anlagebereich nur entlang eines Teils des Umfangs des Penis an, beispielsweise in einem Bereich, welcher das Frenulum umfasst.

Der Aufnahmeraum kann weit geöffnet sein, sodass der Anlagebereich lediglich an einem Teil des Umfangs des Penis, beispielsweise entlang eines Viertels bis zur Hälfte des Umfangs anliegt. Zur Stimulation des Frenulums kann es insbesondere ausreichend sein, wenn der Anlagebereich nur auf der Unterseite des Penis anliegt. Dies erlaubt eine besonders kompakte Ausgestaltung des Applikators und ein einfaches Anlegen, ohne dass der Penis in den Applikator eingeführt werden muss.

In anderen Ausgestaltungen kann der Anlagebereich den Penis weiter umgeben, und beispielsweise an mehr als der Hälfte des Umfangs des Penis bis hin zum gesamten Umfang zur Anlage kommen. Dies kann den Halt des Applikators am Penis verbessern, erfordert jedoch gegebenenfalls das Einführen des Penis in den Applikator.

Der Anlagebereich beziehungsweise der Aufnahmeraum kann durch ein besonders weiches Material gebildet sein, welches sich an die Oberfläche des Penis anschmiegt, beispielsweise ein Silikon oder eine Silikonmischung mit einer geringen Shore-Härte, zum Beispiel kleiner als 5, vorzugsweise kleiner als 3, weiter vorzugsweise kleiner als 2. Es kann auch ein selbstschmierendes Silikon verwendet werden, um den Einsatz von Gleitgel zu vermeiden oder zu reduzieren, insbesondere falls es beabsichtigt ist, den Applikator zu bewegen. Zur Verstärkung der Stimulation bei einer Bewegung des Applikators entlang des Penis, kann der Anlagebereich mit erhabenen Strukturen, wie beispielsweise Noppen, Rippen oder dergleichen versehen sein. Der Druckraum und der Anschluss des Applikators können in einem Teil des Applikators gebildet sein, welcher aus einem steiferen Material gebildet ist und welches beispielsweise eine Schalung um eine weiche Einlage herum bilden kann, welche den Anlagebereich bildet. Als Material kann dafür beispielsweise ein Silikon oder eine Silikonmischung mit einer höheren Shore-Härte, zum Beispiel größer als 20, vorzugsweise größer als 30, weiter vorzugsweise größer als 40, oder ein harter Kunststoff, wie beispielsweise Acrylnitril-Butadien-Styrol (ABS) und dergleichen verwendet werden.

Alternativ kann der Applikator insgesamt aus einem Material bestehen, insbesondere aus einem Stück hergestellt sein. Hierzu eignen sich insbesondere Kunststoffe, welche einerseits nicht zu hart sind und sich zur Anlage am Penis eignen und andererseits zur Ausbildung des Druckraums und des Anschlusses ausreichende Steifigkeit aufweisen, wie Silikone oder geeignete Silikonmischungen. Der Applikator dabei mittels Spritzgussverfahren und insbesondere auch im Zwei- oder Mehrkomponenten-Spritzgussverfahren hergestellt werden, wobei Silikone unterschiedlicher Härte zu einem Teil zusammengefügt werden.

Der Applikator kann des Weiteren eine Halteeinrichtung umfassen, die eingerichtet ist, den Applikatorkörper nach einem Anlegen an dem Penis zu halten. Unter "Halteeinrichtung" werden dabei insbesondere solche Einrichtungen verstanden, welche den Applikator ohne Festhalten durch den Benutzer nach dem Anlegen am Penis an Ort und Stelle halten, ohne dass er herunterfällt. Insbesondere ist die Halteeinrichtung derart ausgebildet, dass sie den Applikator an einer Stelle am Penis hält, bei welcher sich die Öffnung des Druckraums über einem zu stimulierenden Teil des Penis, beispielsweise über dem Frenulum, befindet. Dadurch kann der Applikator "freihändig" benutzt werden, ohne dass der Benutzer den Applikator festhalten muss.

Alternativ kann die Halteeinrichtung dazu dienen, das Halten des Applikators durch den Benutzer zu erleichtern. In diesem Fall kann die Halteeinrichtung beispielsweise auch als "Greifeinrichtung" bezeichnet werden. Es können beispielsweise flexible Flügel vorgesehen sein, welche sich um zumindest einen Teil des Umfangs des Penis legen können und so das Halten des Applikators mit der Hand erleichtern. Es versteht sich, dass andere griffartige Strukturen, die das Festhalten des Applikators erleichtern, ebenfalls denkbar sind.

Die Halteeinrichtung kann sich von dem Applikatorkörper erstrecken und eingerichtet sein, an zumindest einem Teil des Umfangs des Penis zur Anlage zu kommen. Dies ist insbesondere dann vorteilhaft, wenn sich der Anlagebereich des Applikatorkörpers nur über einen Teil des Umfangs des Penis erstreckt. Die Halteeinrichtung kann sich dann vom Applikatorkörper über zumindest einen Teil des Restes oder den gesamten Rest des Umfangs des Penis erstrecken. Die Halteeinrichtung kann beispielsweise Flügel, Bänder, Riemen oder dergleichen aufweisen. Es können gegebenenfalls Mittel zum Befestigen oder Festziehen der Bänder oder Riemen vorgesehen sein. Ferner kann die Halteeinrichtung beispielsweise eine gegebenenfalls flexible, ringartige Struktur, welche sich um den Umfang des Penis herum erstreckt, umfassen. Unabhängig von der Ausgestaltung der Halteeinrichtung kann die Halteeinrichtung lösbar oder fest mit dem Applikatorkörper verbunden sein.

Die Halteeinrichtung kann eingerichtet sein, einen Anpressdruck der Dichteinrichtung an den Penis zu erhöhen. Mit anderen Worten, anstatt den Applikator lediglich festzuhalten, kann die Halteeinrichtung einstellbar sein, um den Anpressdruck der Dichteinrichtung zu verändern. Beispielsweise kann die Halteeinrichtung Mittel zum Festziehen umfassen, wobei beispielsweise ein Innendurchmesser des Aufnahmeraums verkleinert werden kann, sodass sich der Anpressdruck der Dichteinrichtung auf den Penis erhöht. Anstatt eines Mechanismus zum Festziehen kann der Applikator auch eine Andruckeinrichtung, beispielsweise in Form eines oder mehrerer Luftkissen aufweisen, welche aufgepumpt werden können, um den Sitz des Applikators am Penis zu verstärken und den Anpressdruck zu erhöhen.

Es kann beispielsweise vorgesehen sein, dass der Applikator eine äußere Schalung aufweist, welche aus zwei oder mehreren Teilen bestehen kann, die derart relativ zueinander bewegt, beispielsweise verschoben oder verschwenkt werden können, dass sich ein Innendurchmesser des Aufnahmeraums verkleinert. Durch Erhöhen des Anpressdrucks kann die Stimulationswirkung gegebenenfalls erhöht werden und das Risiko des Abrutschens des Applikators kann reduziert werden. Alternativ, insbesondere falls die Halteeinrichtung lediglich als Greifeinrichtung ausgebildet ist, kann der Anpressdruck auch mit der Hand erhöht werden.

Die Halteeinrichtung kann eine Fixiereinrichtung aufweisen, um die Halteeinrichtung in einer gewünschten Stellung zu fixieren. Insbesondere kann auf diese Weise ein gewünschter Anpressdruck gehalten werden, ohne dass der Benutzer den Applikator oder die Halteeinrichtung mit der Hand festhalten muss. Die Fixiereinrichtung kann beispielsweise einen Rastmechanismus umfassen, welcher ein Lösen der Haltevorrichtung nach dem Festziehen verhindert. Es können entsprechende Mittel zum Lösen der Fixiereinrichtung, beispielsweise ein Knopf oder Hebel, vorgesehen sein, sodass der Applikator vom Penis entfernt oder zumindest der Anpressdruck auf den Penis verringert werden kann.

Wie oben bereits erwähnt kann die Stimulationsvorrichtung ein Verbindungsteil zum Verbinden des Applikators mit der Druckfelderzeugungseinrichtung umfassen. Das Verbindungsteil kann einen ersten Anschluss aufweisen, welcher eingerichtet ist, mit dem pneumatischen Ausgang der Druckfelderzeugungseinrichtung fluidisch verbunden zu werden, und einen zweiten Anschluss, welcher eingerichtet ist, mit dem Anschluss des Applikators fluidisch verbunden zu werden, sodass das pneumatische Druckwechselfeld mittels des Verbindungsteils von der Druckfelderzeugungseinrichtung auf den Druckraum des Applikators übertragbar ist.

Das Verbindungsteil kann als separates Teil bereitgestellt sein und sowohl mit dem Applikator als auch der Druckfelderzeugungseinrichtung lösbar verbindbar sein. Dies erlaubt einen modularen Aufbau der Stimulationsvorrichtung, insbesondere falls mehrere, gegebenenfalls verschiedene Applikatoren zur Anwendung kommen sollen. Ferner wird die Reinigung der Stimulationsvorrichtung erleichtert, sodass die Hygiene verbessert werden kann. Das Verbindungsteil kann jedoch mit dem Applikator oder der Druckfelderzeugungseinrichtung oder beiden fest verbunden sein. Das Verbindungsteil kann beispielsweise als Schlauch, insbesondere flexibler Schlauch, als Rohr oder eine andere geeignete Struktur umfassen, welche einen entsprechenden Hohlraum zur Übertragung des Druckwechselfeldes von der Druckerzeugungseinrichtung zum Anschluss des Applikators aufweist.

Ein Schlauch, insbesondere ein längerer Schlauch, erlaubt eine räumliche Distanz zwischen dem Applikator und der Druckfelderzeugungseinrichtung, was eine freihändige Benutzung der Stimulationsvorrichtung erleichtern kann, da die Druckfelderzeugungseinrichtung beispielsweise neben dem Benutzer abgestellt werden kann. Ein kurzes Verbindungsteil oder eine direkte Verbindung der Druckfelderzeugungseinrichtung mit dem Anschluss des Applikators erlaubt ein kompaktes Design der Stimulationsvorrichtung, wobei die Stimulationsvorrichtung im Betrieb dann jedoch möglicherweise aufgrund des höheren Gewichts der Gesamtvorrichtung verglichen mit dem Applikator alleine mit der Hand gehalten werden muss.

Vorteilhaft kommen Steckverbindungen zum Verbinden des Verbindungsteils mit der Druckfelderzeugungseinrichtung und dem Applikator oder zur direkten Verbindung des Applikators mit der Druckfelderzeugungseinrichtung zum Einsatz. Dies erlaubt eine schnelle und einfache Verbindung, sodass die Anwendung der Stimulationsvorrichtung erleichtert wird. Die Steckverbindungen können entsprechende Dichtungen, wie Dichtlippen, O-Ringe oder dergleichen, aufweisen oder von sich aus ausreichend dicht sein, um das Druckwechselfeld im Wesentlichen ohne Verluste zu übertragen. Es versteht sich, dass auch andere Verbindungsmechanismen, wie Schraubverbindungen, Bajonettverbindungen, Schnappverbindungen oder dergleichen möglich sind.

Das Verbindungsteil kann eine Koppelungseinrichtung aufweisen, welche zwischen dem ersten Anschluss und dem zweiten Anschluss angeordnet und eingerichtet ist, das pneumatische Druckwechselfeld zu übertragen und einen Fluidfluss zu verhindern, insbesondere einen Fluidfluss vom zweiten Anschluss zum ersten Anschluss, also vom Applikator zur Druckfelderzeugungseinrichtung. Dadurch kann vermieden werden, dass Wasser, Körperflüssigkeiten oder andere Verschmutzungen von der Öffnung des Druckraums über den Druckraum und den Anschluss des Applikators in das Verbindungsteil und möglicherweise sogar in die Druckfelderzeugungseinrichtung gelangen, was insbesondere durch Kapillarkräfte in einem dünnen Schlauch verursacht werden könnte. Die Koppelungseinrichtung dient somit als Rückflussverschluss.

Vorteilhaft ist die Koppelungseinrichtung zwischen dem ersten und zweiten Anschluss des Verbindungsteils näher am zweiten Anschluss, also näher am Applikator angeordnet, um denjenigen Teil des Verbindungsteils, in den potentiell Flüssigkeiten gelangen können, möglichst klein zu halten. Gegebenenfalls kann die Koppelungseinrichtung direkt am zweiten Anschluss gebildet sein oder den Anschluss zum Applikator bilden. Die Koppelungseinrichtung kann trennbar sein, sodass ein erster Teil des Verbindungsteils mit der Druckfelderzeugungseinrichtung verbunden bleiben kann und ein zweiter Teil des Verbindungsteils, welcher sich zwischen der Koppelungseinrichtung und dem Applikator befindet, separat gereinigt werden kann.

Durch Vorsehen einer Koppelungseinrichtung kann vermieden werden, dass Fluide vom Applikator durch das Verbindungsteil fließen. Gleichzeitig erlaubt die Koppelungseinrichtung in der umgekehrten Richtung eine Übertragung des Druckwechselfeldes von der Druckerzeugungseinrichtung zum Applikator. Dazu kann die Koppelungseinrichtung eine flexible und fluidundurchlässige Membran aufweisen. Insbesondere kann die Membran undurchlässig für Fluide jeglicher Viskosität sein. Es kann jedoch alternativ oder zusätzlich jedoch auch eine semipermeable Membran und/oder eine andere Filtereinrichtung vorgesehen sein, welche zumindest Feststoffe aufhält.

Die Membran kann eine Trennung zwischen zwei Kammern in der Koppelungseinrichtung bilden, wobei sich eine der Kammern auf der der Druckfelderzeugungseinrichtung zugewandten Seite der Membran und die andere der Kammern auf der dem Applikator zugewandten Seite der Membran befindet. Die Kammern erlauben eine Auslenkung der flexiblen Membran, um das Druckwechselfeld zu übertragen. Dazu ist eine Querschnittsfläche der Koppelungseinrichtung vorzugsweise größer als die des Verbindungsteils.

Die Erfindung wird im Folgenden beispielhaft anhand der begleitenden Zeichnungen beschrieben. Die Zeichnungen zeigen zum besseren Verständnis der Erfindung lediglich schematisch bevorzugte Ausführungsbeispiele der Erfindung, wobei die Erfindung nicht auf die gezeigten bevorzugten Ausführungsbeispiele beschränkt ist.
FIG. 1 zeigt einen männlichen Penis.
FIG. 2 zeigt ein Ausführungsbeispiel einer Stimulationsvorrichtung, das dem Wortlaut des Anspruchs 1 nicht entspricht, jedoch zu dessen Verständnis beitragen kann.
FIG. 3 zeigt ein weiteres Ausführungsbeispiel einer Stimulationsvorrichtung, das dem Wortlaut des Anspruchs 1 nicht entspricht, jedoch zu dessen Verständnis beitragen kann.
FIG. 4 zeigt noch ein weiteres Ausführungsbeispiel einer Stimulationsvorrichtung, das dem Wortlaut des Anspruchs 1 nicht entspricht, jedoch zu dessen Verständnis beitragen kann.
FIG. 5 zeigt ein Ausführungsbeispiel einer Druckfelderzeugungseinrichtung, das dem Wortlaut des Anspruchs 1 nicht entspricht, jedoch zu dessen Verständnis beitragen kann.
FIG. 6 zeigt die Druckfelderzeugungseinrichtung aus FIG. 5 in einer perspektivischen Ansicht.
FIG. 7 zeigt eine Antriebseinheit der Druckfelderzeugungseinrichtung.
FIG. 8 zeigt einen elektrischen Antrieb einer Druckfelderzeugungseinrichtung.
FIG. 9 zeigt eine andere Ansicht des elektrischen Antriebs aus FIG. 8.
FIG. 10 zeigt ein Ausführungsbeispiel eines Applikators in verschiedenen Ansichten.
FIG. 11 zeigt ein weiteres Ausführungsbeispiel eines Applikators, angelegt an einen Penis, in verschiedenen Ansichten.
FIG. 12 zeigt ein weiteres Ausführungsbeispiel eines Applikators, angelegt an einen Penis, in verschiedenen Ansichten.
FIG. 13 zeigt ein weiteres Ausführungsbeispiel eines Applikators, angelegt an einen Penis, in verschiedenen Ansichten.
FIG. 14 zeigt ein weiteres Ausführungsbeispiel eines Applikators, angelegt an einen Penis, in verschiedenen Ansichten.
FIG. 15 zeigt ein weiteres Ausführungsbeispiel eines Applikators, angelegt an einen Penis, in verschiedenen Ansichten, das dem Wortlaut des Anspruchs 1 nicht entspricht, jedoch zu dessen Verständnis beitragen kann.
FIG. 16 zeigt ein weiteres Ausführungsbeispiel eines Applikators.
FIG. 17 zeigt eine Stimulationsvorrichtung mit dem Applikator aus FIG. 16.
FIG. 18 zeigt eine Halteeinrichtung eines Applikators.
FIG. 19 zeigt ein weiteres Ausführungsbeispiel eines Applikators und dessen Halteeinrichtung.
FIG. 20 zeigt ein weiteres Ausführungsbeispiel eines Applikators.
FIG. 21 zeigt ein weiteres Ausführungsbeispiel eines Applikators.
FIG. 22 zeigt eine Koppelungseinrichtung für ein Verbindungsteil zwischen einem Applikator und einer Druckfelderzeugungseinrichtung.
FIG. 23 zeigt ein Ausführungsbeispiel einer Steckverbindung.

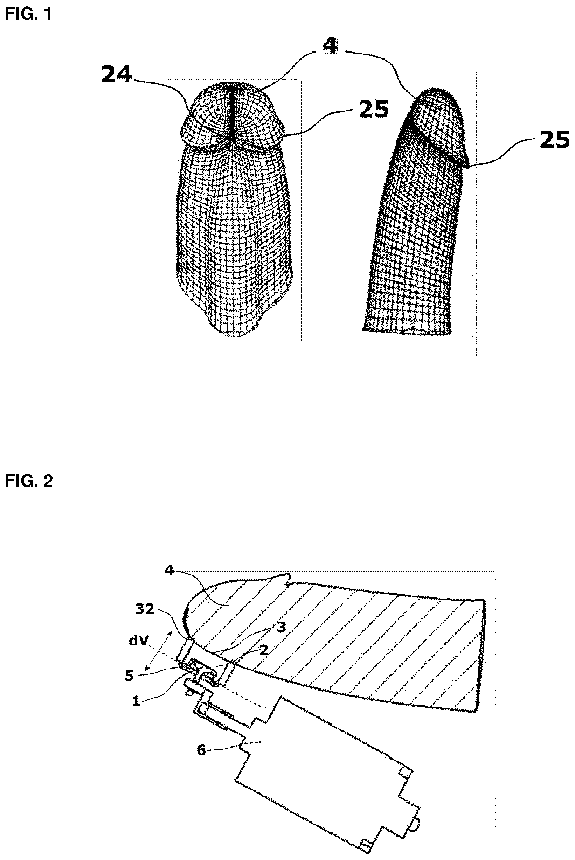

Zum besseren Verständnis der Erfindung werden mit Bezug auf FIG. 1 relevante Aspekte der Anatomie eines menschlichen, männlichen Penis (beziehungsweise Glieds), insbesondere hinsichtlich einer gewünschten Stimulation mittels einer Stimulationsvorrichtung gemäß der Erfindung erläutert. Die Eichel 4 und das Frenulum 24 des männlichen Glieds gehören zu den stärksten erogenen Zonen des männlichen Körpers, wobei eine geeignete Stimulation beziehungsweise Reizung zu sexueller Erregung führen beziehungsweise sexuelle Erregung steigern kann. Die sexuelle Erregung kann zum männlichen Orgasmus und Ejakulationsreflex führen. Auch kann durch einen Unterdruck die Durchblutung des stimulierten Bereichs gefördert und durch einen Überdruck eine stimulierende Kraft aufgebracht werden.

Das Frenulum 24 und die Eichelkranzfurche 25, d.h. der Übergang zwischen Eichel 4 und Penisschaft, sind besonders sensibel für die genannte Stimulation beziehungsweise Reizung.

Mit einer Stimulationsvorrichtung gemäß der Erfindung, insbesondere mit einem entsprechenden erfindungsgemäßen Applikator ist es möglich, die Eichel 4 des männlichen Glieds, insbesondere das Frenulum, im ständigen Wechsel von Über- und Unterdrücken in geeigneter Stärke zu durchbluten und zu stimulieren, so dass eine in ihrer Frequenz und Amplitude geeignete Druckfeldstimulation zur sexuellen Erregung des Mannes, im besten Fall bis zum männlichen Orgasmus, führt.

Um ein zur sexuellen Stimulation geeignetes Druckwechselfeld (im Folgenden auch einfach Druckfeld genannt) mit ausreichender Stärke aufbauen zu können, ist wie nachfolgend erläutert ein Applikator mit einer Öffnung vorgesehen, um ein mittels einer Druckfelderzeugungseinrichtung erzeugtes Druckwechselfeld direkt auf die Haut des Penis zu applizieren. Der Applikator ist an die Anatomie des männlichen Glieds angepasst und insbesondere zum Auflegen über das Frenulum 24 und die Eichelkranzfurche 25, sowie auch insbesondere zur Aufnahme des Frenulums 24 konzipiert. Durch Vorsehen einer Dichteinrichtung kann das zur Stimulation erforderliche Druckwechselfeld im ausreichenden Maße aufgebaut werden, indem ein Druckraum des Applikators gegen die Umgebung abgedichtet wird.

Die Stimulationsvorrichtung ist derart gestaltet, dass die Stimulation nicht zu einem Austrocknen der Schleimhäute führt, was beispielsweise durch einen ständigen Luftaustausch mit der Umgebung passieren würde. Außerdem ist die Vorrichtung für eine hygienische Benutzung gestaltet, d.h. schwer zugängliche und damit schwer zu reinigende Hohlräume, in denen sich beispielsweise Körperflüssigkeit sammeln können, wie zum Beispiel in Ventilen, werden vermieden. Darüber hinaus kann sich die Temperatur des in dem Druckraum des Applikators eingeschlossenen, relativ kleinen Luftvolumens schnell der Körpertemperatur angleichen, sodass eine angenehme Benutzung gewährleistet wird. Zudem ist die Stimulationsvorrichtung einfach im Aufbau und damit einfach in der Benutzung, sodass eine Ablenkung des Nutzers bei der Benutzung und der Aufwand zur Benutzung minimiert sind.

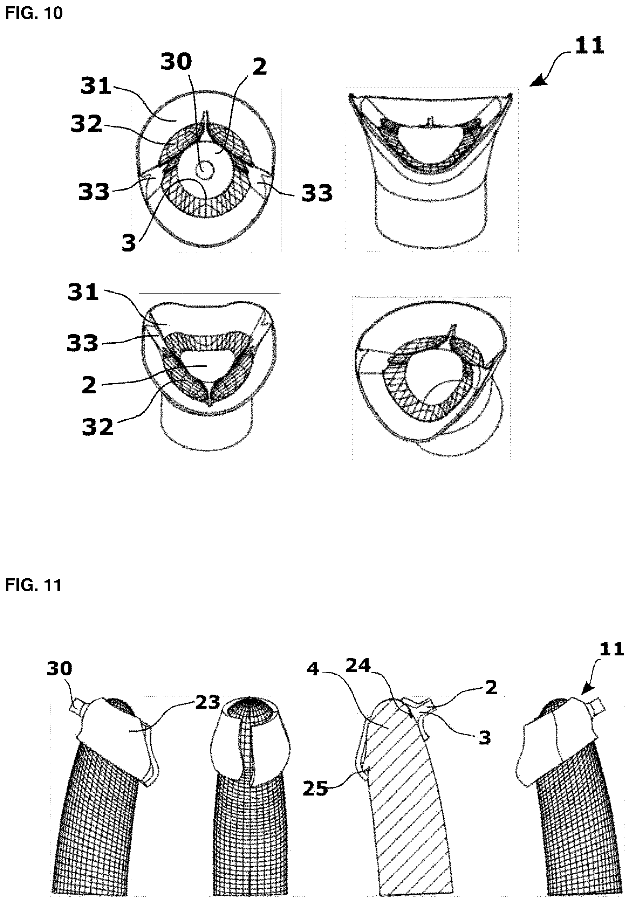

Als geeignete sexuelle Stimulation der Eichel 4 des männlichen Glieds, bestenfalls bis zum Orgasmus, ist insbesondere eine vergleichsweise hochfrequente Wechselfrequenz zwischen 5 Hz bis 250 Hz und eine Druckdifferenz von 20 mbar bis 800 mbar um den Umgebungsdruck von in etwa 1 bar, vorzugsweise symmetrisch um den Umgebungsdruck, vorteilhaft. Ein derartiges Druckwechselfeld bestehend aus einem Wechsel von Unter- und Überdrücken mit einer Wechselfrequenz zwischen 5 Hz bis 250 Hz und Druckdifferenzen von 20 mbar bis 800 mbar kann mittels einer Druckfelderzeugungseinrichtung generiert werden, die in den FIG. 2 bis 9 schematisch dargestellt ist. Verschiedene Ausführungsbeispiele von Applikatoren sind in den FIG. 10 bis 21 dargestellt. Es versteht sich, dass die verschiedenen Ausführungsbeispiele oder Aspekte der verschiedenen Ausführungsbeispiele beliebig kombiniert werden können.

In FIG. 2 ist ein erstes Ausführungsbeispiel einer Stimulationsvorrichtung mit einer Druckfelderzeugungseinrichtung 1 dargestellt. Die Druckfelderzeugungseinrichtung 1 weist zumindest eine Wand 5 oder Membran auf, welche aus einem elastischen Material, beispielsweise Silikon oder Gummi, besteht, wobei diese flexible Wand 5 mittels eines Antriebs 6 ausgelenkt wird, um eine positive und negative Volumenänderung dV in der Druckfelderzeugungseinrichtung 1 zur Erzeugung des Druckfelds, insbesondere Druckwechselfelds aus Unterdruck- und Überdruckphasen, zu bewirken. Es ist ein Druckraum 2 vorgesehen, in welchem sich das durch Auslenkung der Wand 5 erzeugte Druckwechselfeld aufbaut. Die flexible Wand 5 dient insbesondere nur zur Erzeugung des Druckwechselfeldes im Druckraum 2 und ist von der Öffnung 3 beabstandet, d.h. kontaktiert im Betrieb (also insbesondere in jeder ausgelenkten Stellung) nicht den Penis. Der Druckraum 2 kann in Strömungsrichtung einen unveränderlichen Querschnitt aufweisen, so dass der Druckraum im Wesentlichen zylindrisch ausgebildet ist.

Der Druckraum 2 weist eine Öffnung 3 auf, welche derart gestaltet ist, dass der Druckraum 2 beim Aufsetzen auf einen zu stimulierenden Bereich des Penis, wie die Eichel 4 oder insbesondere das Frenulum 24, gegen die Umgebung, d.h. gegen Bereiche außerhalb des Druckraums, abdichtet oder annähernd abgedichtet wird, sodass das Druckwechselfeld aufgebaut werden kann. Dazu ist eine Dichteinrichtung 32 vorgesehen, welche sowohl zur Abdichtung gegen Unterdrücke als auch Überdrücke relativ zum Umgebungsdruck eingerichtet ist. Sie umgibt die Öffnung 3 und kann an die Eichel 4 dichtend angelegt werden, insbesondere derart, dass das Frenulum 24 in der Öffnung 3 zumindest teilweise aufgenommen wird. Die Fläche der Öffnung 3 ist gleich der Querschnittsfläche des Druckraums 2. Eine Stimulation des Frenulums 24 kann einen besonders starken Effekt hinsichtlich sexueller Erregung bewirken.

Annähernd dichtendes Aufsetzen über die männliche Eichel 4 bedeutet dabei insbesondere, dass mittels der Druckfelderzeugungseinrichtung 1 ein zumindest weitgehend abgeschlossenes Strömungssystem ausgebildet wird. In dem Strömungssystem werden Medienströmungen erzeugt, insbesondere pneumatische Strömungen, d.h. Luftströmungen, die zeitlich abwechselnd auf die männliche Eichel 4 gerichtet (Überdruck) und von der männlichen Eichel 4 weg gerichtet (Unterdruck) sind. Es versteht sich, dass dies gleichermaßen für jeden anderen gewünschten zu stimulierenden Bereich des Penis möglich ist. In diesem abgeschlossenen Strömungssystem wird einen Abtransport von Körperflüssigkeit aus der Druckfelderzeugungseinrichtung 1 weitgehend vermieden.

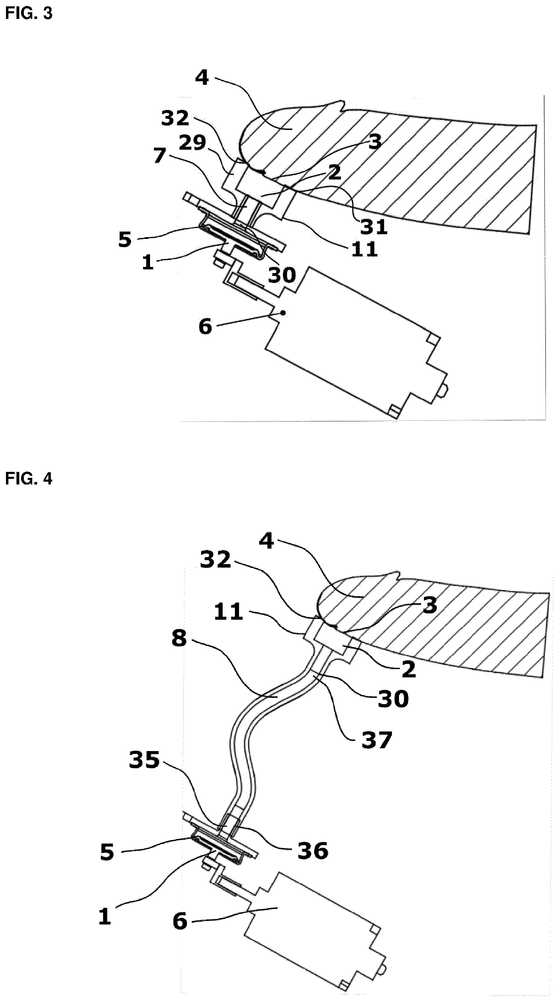

In FIG. 3 ist ein Ausführungsbeispiel einer Stimulationsvorrichtung dargestellt, in welcher die Druckfelderzeugungseinrichtung 1 mit einem Applikator 11 verbunden ist. Der Applikator 11 weist einen Applikatorkörper 29 auf, in welchem der Druckraum 2 gebildet ist. Der Applikator 11 weist ferner einen Anschluss 30 auf, welcher über einen Fluidkanal 7 mit dem Druckraum 2 verbunden ist, um ein mittels der Druckfelderzeugungseinrichtung 1 erzeugtes Druckwechselfeld durch die Öffnung 3 auf den Penis zu übertragen. Der Anschluss 30 liegt bezüglich einer Längsachse des Druckraums 2 der Öffnung 3 gegenüber. Die flexible Membran 5 liegt auf einer dem Druckraum 2 gegenüberliegenden Seite des Anschlusses 30 außerhalb des Druckraums 2 und ist insbesondere durch den Fluidkanal 7 von dem Druckraum 2 beabstandet. Im Anlagebereich 31 ist um die Öffnung 3 umlaufend eine Dichteinrichtung 32, beispielsweise eine Dichtlippe oder -wulst ausgebildet, um den Druckraum 2 gegen die Umgebung abzudichten.

FIG. 4 zeigt ein Ausführungsbeispiel, in welchem der Applikator 11 über ein Verbindungsteil 8 in Form eines Schlauchs mit der Druckfelderzeugungseinrichtung 1 verbunden ist, um das mittels der Druckfelderzeugungseinrichtung 1 erzeugte Druckwechselfeld auf den Applikator 11 zu übertragen und es durch die Öffnung 3 auf den Penis aufzubringen. Insbesondere ist ein erster Anschluss 36 des Verbindungsteils 8 mit einem pneumatischen Ausgang 35 der Druckfelderzeugungseinrichtung 1 verbunden, während ein zweiter Anschluss 37 des Verbindungsteils 8 mit dem Anschluss 30 des Applikators 11 verbunden ist. Dies kann beispielsweise über Steckverbindungen erfolgen, wie unten detaillierter beschrieben. Das Verbindungsteil 8 kann abschnittsweise oder vollständig flexibel ausgestaltet sein.

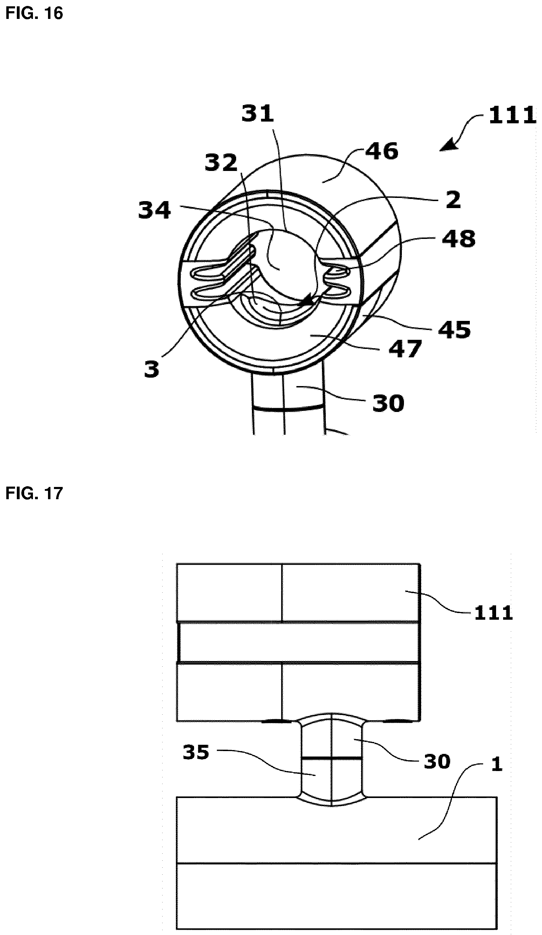

Die Konfiguration der Stimulationsvorrichtung mit einem Schlauch als Verbindungsteil 8 kann der einfachen Benutzung der Stimulationsvorrichtung dienen, da der Benutzer nur den Applikator 11 mit der Hand halten muss. Gegebenenfalls kann dies auch ohne Schlauch bei geeigneter Gestaltung der Form der Stimulationsvorrichtung gegebenenfalls mit einem kurzen Verbindungstück möglich sein (vgl. beispielsweise FIG. 17). Bei gleichzeitigem Vorsehen einer entsprechenden Halteeinrichtung, wie unten detaillierter beschrieben, kann so auch insbesondere eine "freihändige" Benutzung möglich sein, da die Druckfelderzeugungseinrichtung 1 (beispielsweise neben oder auf dem Benutzer) abgelegt werden kann.

Eine Querschnittsänderung des Druckraums 2 beziehungsweise eines Druck- oder Strömungssystems, welches neben dem Druckraum 2 auch den Fluidkanal 7, das Verbindungsteil 8 und/oder andere dem Druckwechselfeld ausgesetzte Hohlräume umfassen kann, kann sich auf die Strömungsgeschwindigkeit des Mediums (insbesondere Luft) auswirken, d.h. eine Querschnittsverengung bedeutet eine Strömungsbeschleunigung und eine Querschnittserweiterung entsprechend eine Strömungsverzögerung.

Da der Druckraum 2 annähernd dichtend auf den Penis aufgesetzt und damit ein zumindest weitgehend abgeschlossenes Strömungssystem ausgebildet wird, findet so gut wie kein Luftaustausch mit der Umgebung statt, weshalb der Abtransport von Körperflüssigkeit aus der Druckfelderzeugungseinrichtung vermieden wird und die Stimulation nicht zum Austrocknen der Schleimhäute führt. Die Temperatur des im abgeschlossenen System eingeschlossenen Luftvolumens gleicht sich aufgrund des relativ kleinen Volumens schnell der Körpertemperatur an. Des Weiteren kann die Stimulationsvorrichtung ohne Ventile auskommen, was eine hygienische Nutzung erleichtert.

Auch im Hinblick auf eine hygienische Nutzung ist ein durch einen Anlagebereich 31 definierter Aufnahmebereich oder Aufnahmeraum 34 des Applikators 11 im Bereich der Spitze der Eichel 4 vorzugsweise offen. Somit ist bei Benutzung der erfindungsgemäßen Stimulationsvorrichtung, insbesondere aufgrund der kompakten Form des Applikators 11, welcher vorzugsweise zur Stimulation des Frenulums 24 eingerichtet ist und somit nur einen kleinen Teil des Penis gegen die Umgebung abdichten muss, der Austritt des Ejakulats beim Orgasmus möglich. Eine Reinigung wird somit vereinfacht. Dies schafft eine Stimulationsvorrichtung, welche insbesondere im Vergleich zu herkömmlichen geschlossenen Stimulationsvorrichtungen hinsichtlich Hygiene verbessert ist.

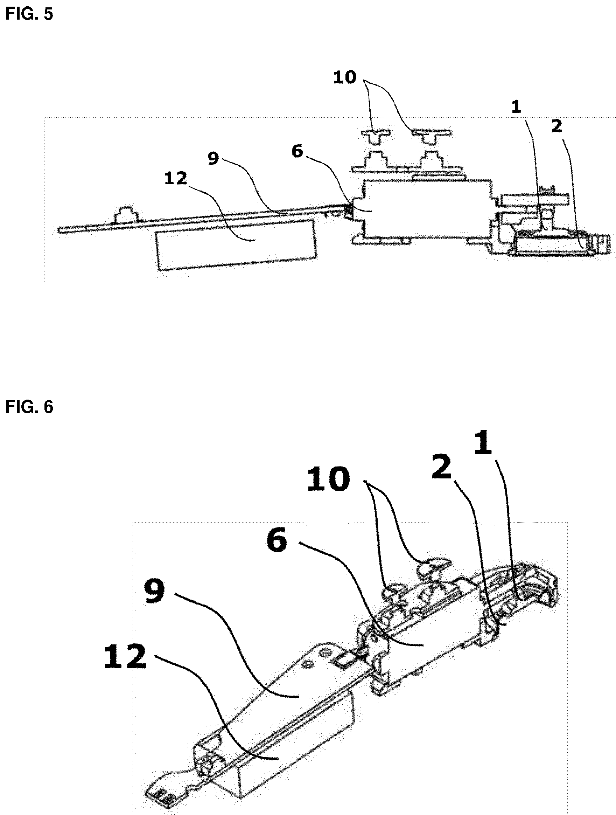

Wie in den FIG. 5 und 6 dargestellt ist, weist die Stimulationsvorrichtung neben der Druckfelderzeugungseinrichtung 1 eine Steuereinrichtung 9 auf, welche eine Antriebseinheit 6 ansteuern kann und in der die Modulation des Druckfelds vorgespeichert sein kann. Die Steuereinrichtung 9 weist zumindest ein Bedienelement 10 auf, wobei die jeweilige Modulation des Druckfeldes mittels des Bedienelements 10 veränderbar sein kann. Außerdem besitzt die Stimulationsvorrichtung ein Gehäuse (nicht dargestellt), welches die Steuereinrichtung 9, die Antriebseinheit 6, die Druckfelderzeugungseinrichtung 1 und eine interne Batterie 12 umfassen kann, wobei die Stimulationsvorrichtung vorzugsweise als ein tragbares Handgerät ausgebildet ist. Die Steuereinrichtung 9 ermöglicht mittels eines Bedienelements 10 ein Stimulationsmuster aus den Stimulationsmustern der Steuereinrichtung 9 einzustellen, wobei die Antriebseinheit 6 entsprechend dem eingestellten Stimulationsmuster angesteuert wird.

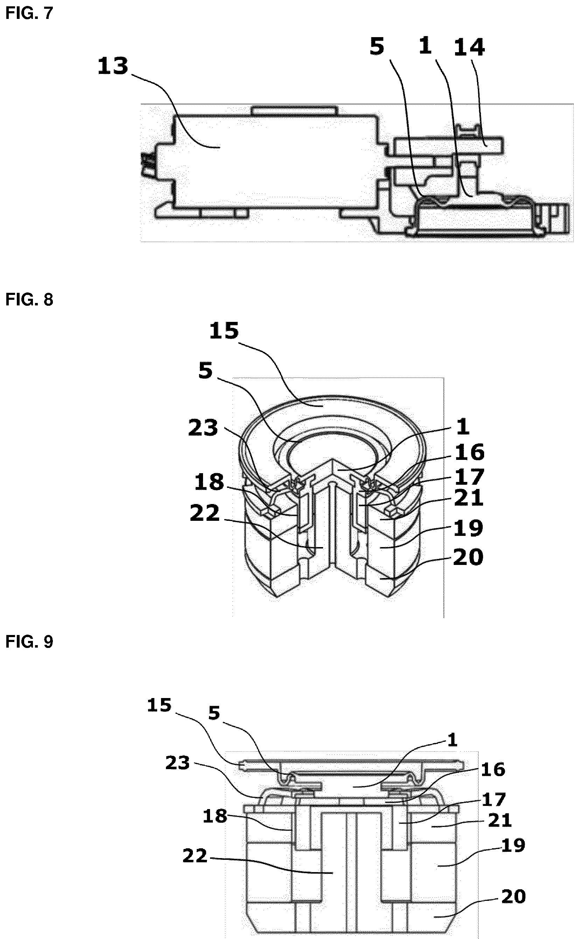

Die an die flexible Wand 5 der Druckfelderzeugungseinrichtung 1 gekoppelte Antriebseinheit 6 kann beispielweise aus einem rotierenden Elektromotor 13 mit mechanischer Übersetzung bestehen. Die mechanische Übersetzung der Rotation des Elektromotors 13 in eine translatorische Bewegung der flexiblen Wand 5 der Druckfelderzeugungseinrichtung 1 kann beispielsweise mittels eines Exzenters 14 erfolgen, wie schematisch in FIG. 7 gezeigt.

In FIG. 7 ist schematisch die Antriebseinheit 6 in Form eines rotierenden Elektromotors mit mechanischer Übersetzung und Koppelung an die flexible Wand 5 der Druckfelderzeugungseinrichtung dargestellt. Mittels des durch den rotierenden Elektromotor 13 zugeführten Steuerstroms, beispielsweise in Form von Gleichstrom, wird die Drehzahl des Elektromotors 13 und damit letztlich die Frequenz der flexiblen Wand 5 variiert beziehungsweise gesteuert. Die flexible Wand 5 kann eine Sicke aufweisen, die den Hüben der flexiblen Wand 5 mechanisch weitestgehend ohne mechanische Spannungen folgt. Der Hub der flexiblen Wand 5 ist durch den definierten Exzenterweg festgelegt. Der feste Kolbenhub bedeutet eine feste Verringerung und Vergrößerung des Kammervolumens dV und damit entsprechend eine feste Druckerhöhung beziehungsweise Druckverringerung, d.h. eine annähernd feste Amplitude des wechselnden Über- und Unterdrucks.

Alternativ kann die an die flexible Wand 5 der Druckfelderzeugungseinrichtung 1 gekoppelte Antriebseinheit 6 aus einem linearen Elektromotor 15 bestehen, wie in den FIG. 8 und 9 dargestellt. Beim dem in FIG. 8 und 9 dargestellten elektromagnetischen Wandler wird die flexible Wand 5 verbunden mit einem Träger 16 durch mit mindestens einer daran befestigten Schwing- beziehungsweise Tauchspule 17 entsprechend der Spulenspeisung mittels des Steuerstroms im Luftspalt 18 hin und her bewegt.

Die flexible Wand 5 der Druckfelderzeugungseinrichtung 1 ist an einem Träger 16 befestigt. Die flexible Wand 5 kann eine Sicke aufweisen, die den Hüben der flexiblen Wand 5 mechanisch weitestgehend ohne mechanische Spannungen folgt. Um den Träger 16 ist eine Schwingspule 17 gewickelt, welche im Betrieb durch den Steuerstrom aus einer Steuereinheit gespeist wird. Die Schwingspule 17 besteht aus elektrischen Leitern aus einem elektrisch möglichst leitfähigen Material (bevorzugt Kupfer), die mit einem elektrisch isolierenden Lack gegeneinander und gegen den Träger 16 isoliert sind. Das Magnetfeld wird durch mindestens einen Permanentmagneten 19 bevorzugt in Ringform erzeugt.

Der magnetische Fluss wird beispielsweise mittels einer hinteren Polplatte 20 (bevorzugt in Zylinderform) über die obere Polplatte 21 (bevorzugt in Ringform) über den bevorzugt ringförmigen Luftspalt zum zylinderförmigen Polkern 22 geführt. Die hintere Polplatte 20 und die obere Polplatte 21 sind ebenso wie der Polkern 22 aus magnetisch hochpermeablem Material (bevorzugt einer weichmagnetischen Werkstofflegierung). Alternativ kann auch ein zylinderförmiger Permanentmagnet anstelle des Polkerns 22 und entsprechend ein Ringpol anstelle des Permanentmagneten 19 verwendet werden.

Der Träger 16 mit der Schwingspule 17 wird konstruktiv durch mindestens eine Halterung beziehungsweise Aufhängung 23 (bevorzugt aus Kunststoff, Textilgewebe oder Papier) im Luftspalt 18 zentriert und geführt, um Taumelbewegungen der Schwingspule 17 zu verhindern. Die Halterung beziehungsweise Aufhängung 23 wird an einem Rahmen befestigt.

Zur Bewegung der flexiblen Wand 5 wird die Schwingspule 17 mit einem Steuerwechselstrom aus einer Steuereinheit gespeist. Die Schwingspule 17 wird je nach Stromrichtung beziehungsweise Strompolarität im Magnetfeld des Luftspalts 18 durch die Lorentz-Kraft nach oben oder nach unten bewegt. Der Hub der Auslenkung der Schwingspule 17 wird durch die Amplitude des Steuerstroms bestimmt. Die Frequenz des Wechselstroms entspricht der Frequenz der Schwingspulenbewegung und damit der Frequenz der Kolben- beziehungsweise Membranbewegung. Die Frequenz und der Hub der Schwingspule 17 und damit die Bewegung der flexiblen Wand 5 können somit vergleichsweise einfach durch die Stromfrequenz und Stromamplitude unabhängig voneinander gesteuert werden. Aufgrund der direkten Übertragung ist ein erweiterter Frequenzbereich mit diesem Prinzip von unter 1 Hz bis zu mehreren Hundert Hz möglich. Ein Gleichstrom aus einer Batterie oder einem Akkumulator wird in ein Wechselstromsignal umgerichtet.

Entscheidend für den Aufbau des gewünschten Druckwechselfeldes, insbesondere mit den genannten Unter- und Überdrücken in der Größenordnung von Druckdifferenzen von 20 mbar bis 800 mbar ist die Abdichtung des Druckraums 2 gegen die Umgebung, mit anderen Worten das Aufliegen des Anlagebereichs 31 mit der Dichteinrichtung 32 auf dem Penis, insbesondere der Eichel 4, und das Ausbilden ein zumindest weitgehend abgeschlossenes Strömungssystems. Zu diesem Zwecke ist der Anlagebereich 31 und insbesondere die Dichteinrichtung 32 bezüglich Form und Material geeignet ausgestaltet, wie im Folgenden anhand bevorzugter Ausführungsbeispiele beschrieben. Des Weiteren kann eine für den Aufbau der genannten Unter- und Überdrücken ausreichende Anpresskraft auf die Dichteinrichtung 32 aufgebracht werden, wie ebenfalls im Folgenden anhand bevorzugter Ausführungsbeispiele beschrieben wird. Es versteht sich, dass relevante Merkmale insbesondere hinsichtlich der Übertragung des Druckwechselfeldes auf den Penis allen Ausführungsbeispielen gemein ist und lediglich zur Vermeidung von Wiederholungen nicht bezüglich jedes Ausführungsbeispiels beschrieben wird.

In FIG. 10 ist ein Ausführungsbeispiel eines Applikators 11 dargestellt, welcher neben der Dichteinrichtung 32 anatomisch geformte Vorsprünge 33 im Anlagebereich 31 aufweist. Außerdem ist der Anlagebereich 31 derart ausgestaltet, dass er eine die Dichtung unterstützende Oberflächenform aufweist. Es ist eine Dichteinrichtung 32 vorgesehen, welche die Öffnung 3 des Druckraums 2 des Applikators 11 umgibt. Die Dichteinrichtung 32 ist als abgerundeter nachgiebiger Wulst um die Öffnung 3 herum ausgebildet, welcher sich beim Anlegen des Applikators 11 an den Penis der Form des Penis anpassen kann.

Darüber hinaus sind die Vorsprünge 33 vorgesehen, welche im Wesentlichen dem Verlauf der Eichelkranzfurche 25 folgen. Dies kann die Dichtwirkung der Dichteinrichtung 32 unterstützen, sodass die Vorsprünge 33 auch als Teil der Dichteinrichtung 32 angesehen werden können. Die Vorsprünge 33 können aufgrund ihrer anatomischen Form das korrekte Positionieren des Applikators 11 auf dem Penis erleichtern, insbesondere derart, dass das Frenulum 24 zumindest teilweise in dem Druckraum 2 aufgenommen wird. Das Material der Dichteinrichtung 32 ist vorzugsweise weich, beispielsweise ein weiches Silikon oder Gummi. Die Vorsprünge 33 können aus demselben Material oder aus einem anderen, beispielsweise härteren oder weicheren Material gebildet sein. Die Öffnung 3 des Druckraums 2 ist derart ausgebildet, dass sie das Frenulum 24 zumindest teilweise aufnehmen kann, sodass das Druckwechselfeld im Druckraum 2 direkt auf das Frenulum 24 wirken kann.

Gemäß einem Ausführungsbeispiel kann die für eine Abdichtung nötige Anpresskraft durch den Handdruck des Nutzers selbst aufgebracht werden. In FIG. 11 ist schematisch ein solcher Applikator 11, angelegt an einen Penis, dargestellt, bei welchem der Druckraum 2 durch die mittels des Handdrucks aufgebrachte Anpresskraft gegen die Eichel 4 abgedichtet wird. Es ist eine Halteeinrichtung beziehungsweise Greifeinrichtung in Form von flügelartigen Fortsätzen 23 gebildet, welche der Nutzer weitgehend flexibel auf dem Penis beziehungsweise der Eichel 4 positionieren kann. Auf diese Weise wird die Dichteinrichtung gegen den Penis gedrückt. Die Öffnung 3 ist ausgebildet, das Frenulum 24 und Teile oder den gesamten Eichelkranz 25 aufzunehmen. In diesem Ausführungsbeispiel erweitert sich der Druckraum 2 zur Öffnung 3 hin, wie in der Schnittansicht in FIG. 11 zu erkennen ist.

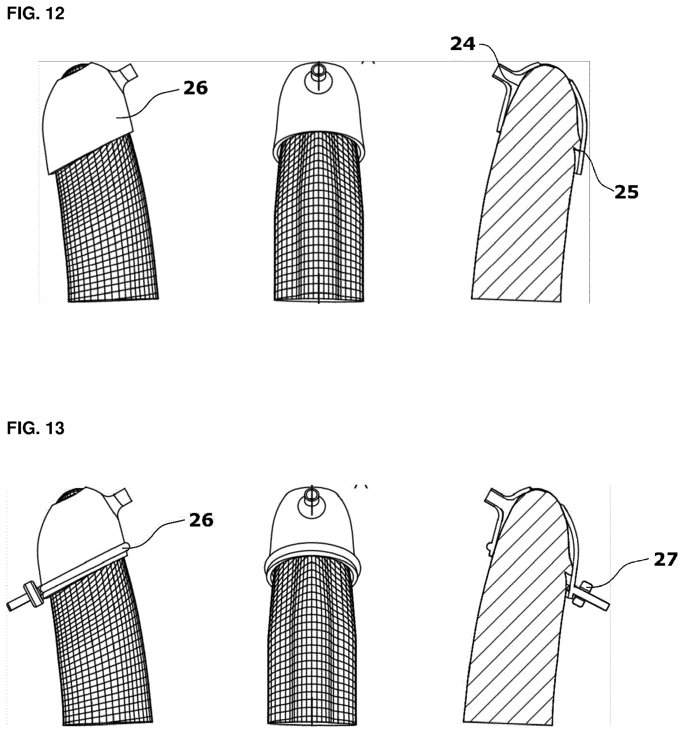

Gemäß einem weiteren Ausführungsbeispiels, wie in FIG. 12 dargestellt, kann die nötige Anpresskraft beispielsweise durch eine im Wesentlichen ringförmige Halteeinrichtung 26 erreicht werden, wobei die bei elastischer Verformung des Rings 26 auftretenden Materialeigenspannungen auf den Penis und auf die Dichteinrichtung wirken. Vorteilhaft ist die Halteeinrichtung 26 im Bereich der Spitze der Eichel 4 offen. Denkbar ist jedoch auch eine an der Spitze geschlossene kappenartige Struktur, welche wie die ringförmige Halteeinrichtung 26 elastisch verformbar ist und den Applikator 11 so auf der Eichel 4 festhält. Die Öffnung 3 des Druckraums 2 wird bei dem in FIG. 12 dargestellten Ausführungsbeispiel nach Dehnung und Aufsetzen der ringförmigen Halteeinrichtung 26 gegen die Eichel 4 gedichtet, wobei die Öffnung 3 das Frenulum 24 und Teile oder den gesamten Eichelkranz 25 aufnimmt.

Die in FIG. 12 gezeigte ringförmige und elastisch verformbare Halteeinrichtung 26 kann durch einen Riemen mit einem Straffungsmechanismus 27 verstärkt werden wie FIG. 13 dargestellt. Hierbei wird die Abdichtung durch die mittels Materialeigenspannung bei elastischer Verformung und zusätzlich durch eine mittels eines Riemens aufgebrachte Kraft gegen die Eichel 4 des männlichen Glieds erreicht.

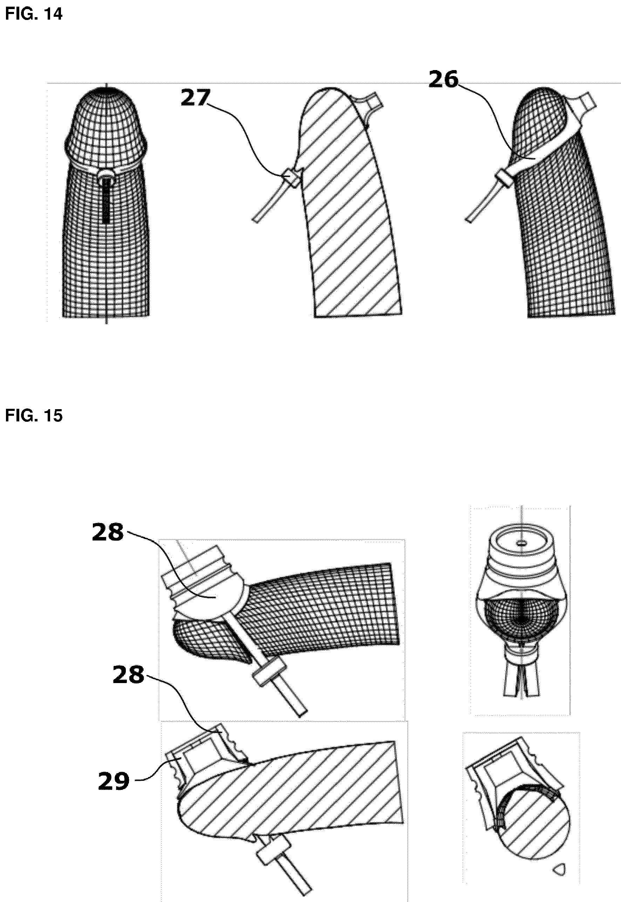

Alternativ kann gemäß einem weiteren Ausführungsbeispiel die nötige Anpresskraft allein durch eine riemen- oder bandförmige Halteeinrichtung 26 mit einem Fixier- oder Straffungsmechanismus 27 aufgebracht werden wie in FIG. 14 dargestellt. Der Riemen lässt sich besonders einfach am Eichelkranz 25 fixieren und damit positionieren. Darüber hinaus lässt sich diese Halteeinrichtung 26 des Applikators 11 besonders einfach unterschiedlichen Durchmessern des männlichen Gliedes im Bereich der Eichel 4 anpassen.

In einer weiteren Variante des Applikators wie in FIG. 15 gezeigt kann die Halteeinrichtung separat vom Applikatorkörper 29 ausgebildet sein. Dadurch kann durch Wahl einer passenden Halteeinrichtung die Abdichtung auch bei unterschiedlichen Glied- beziehungsweise Eicheldurchmessern verbessert und die Benutzung im Hinblick auf An- und Ablegen der Stimulationsvorrichtung sowie der Reinigung vereinfacht werden. Diese Mehrstückigkeit wird in FIG. 15 beispielhaft am Riemendichtungssystem aufgezeigt. Ein von dem Applikatorkörper 29 separater Haltekörper 28 mit Riemen wird über dem Applikatorkörper 29 angeordnet, so dass die nötige Anpresskraft auf die Dichteinrichtung aufgebracht werden kann. Die beiden Teile können aus dem gleichen Material oder aus unterschiedlichen Materialien sein.

Die in den FIG. 10 bis 15 gezeigten Ausführungsbeispiele sind insbesondere für eine stationäre oder unbewegte Benutzung der Stimulationsvorrichtung beziehungsweise des Applikators 11 konzipiert. Mit anderen Worten, der Applikator 11 kann insbesondere hinsichtlich Form, insbesondere des Anlagebereichs 31 und der Dichteinrichtung 32 derart gestaltet sein, dass er auf den Penis aufgelegt und gegebenenfalls mit einer Halteeinrichtung befestigt wird, jedoch ohne bewegt zu werden. Dabei wird zur Stimulation das Druckwechselfeld über die Öffnung 3 insbesondere auf das Frenulum 24 appliziert.

Es kann jedoch gewünscht sein, dass der Applikator über den Penisschaft, die Eichelkranzfurche 25, den Eichelkranz und die Eichel 4 bewegt werden kann. Gleichzeitig soll jedoch beim Aufbringen eines Anpressdrucks beziehungsweise einer Druckkraft eine Dichtverbindung hergestellt werden, um die Stimulation wie in den anderen Ausführungsbeispielen durch Applizieren des Druckwechselfeldes über die Öffnung 3 zu erreichen. Der Applikator gemäß den Ausführungsbeispielen der FIG. 16 bis 21 ist derart gestaltet, dass er insbesondere durch Lösen oder zumindest Lockern des Anpressdrucks entlang des Penisschafts bewegt werden kann. Eine Bewegung kann jedoch auch bei Aufrechterhalten des Anpressdrucks möglich sein.

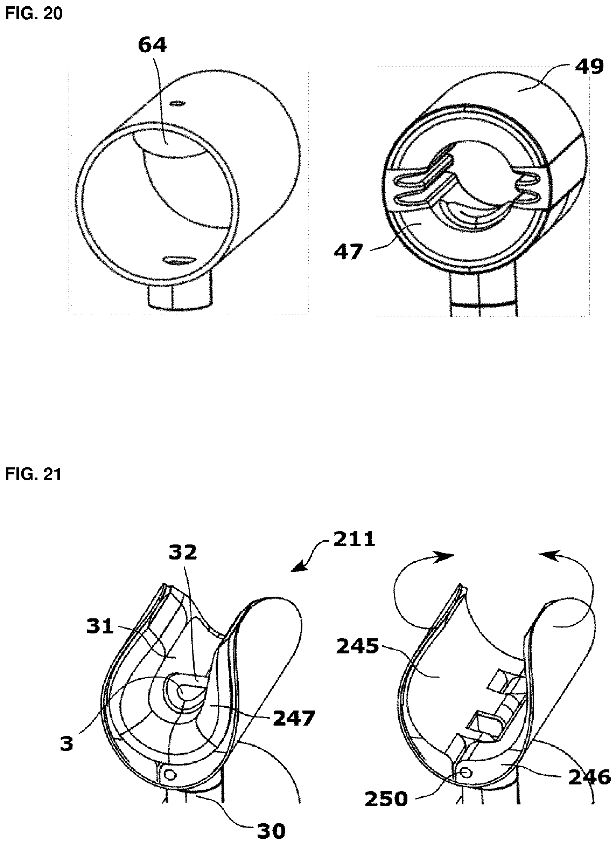

Die Dichteinrichtung 32 ist dazu vorzugsweise derart gestaltet, dass sie sich einerseits sich aufgrund der auferlegten Anpresskraft und wulstartigen Lippen um die Öffnung 3 des Druckraums 2 an die Oberfläche der Eichelfurche und andere Bereiche am Penis anpasst und dichtend anlegt und andererseits bei Lösen oder Lockern des Anpressdrucks oder auch bei Aufbringen oder Aufrechterhalten eines Anpressdrucks bewegen lässt. Vorteilhaft erstreckt sich dazu der Anlagebereich 31 um den gesamten Umfang des Penis herum und definiert einen umlaufend geschlossenen Aufnahmeraum 34 (FIG. 16 bis 20), kann jedoch auch am Umfang offen sein (FIG. 21). Auch die im Folgenden beschriebenen Halteeinrichtungen sind einerseits für eine Fixierung des Applikators am Penis geeignet, können jedoch entsprechend gelockert oder gelöst werden, um eine Bewegung des Applikators entlang des Penisschafts zu erlauben. Bei einer Bewegung kann das Druckwechselfeld weiterhin erzeugt oder abgeschaltet werden. Die Dichteinrichtung kann dabei eine Gleitdichtung bilden, sodass auch bei Bewegung zumindest eine teilweise bis hin zur gleichen oder annähernd gleichen Dichtwirkung wie beim stationären Betrieb erreicht werden kann und das Druckwechselfeld im Druckraum 2 aufgebaut und durch die Öffnung 3 auf den Penis appliziert werden kann.

In FIG. 16 ist ein Ausführungsbeispiel eines Applikators 111 dargestellt, welches einen umlaufend zusammenhängenden Anlagebereich 31 und damit einen umlaufend geschlossenen Aufnahmeraum 34 aufweist, in welchen der Penis eingeführt werden kann. Der Applikator 111 weist analog zu den oben beschriebenen Ausführungsbeispielen einen Anschluss 30 auf, über den ein von einer Druckfelderzeugungseinrichtung 1 erzeugtes Druckwechselfeld in einen Druckraum 2 des Applikators 111 und schließlich über die Öffnung 3 auf einen zu stimulierenden Teil des Penis, wie beispielsweise das Frenulum, aufgebracht werden kann. Zum Abdichten des Druckraums 2 gegenüber der Umgebung ist eine Dichteinrichtung 32 um die Öffnung 3 im Anlagebereich 31 ausgebildet.

Der Applikator 111 weist eine steife Schalung auf, welche einen ersten Teil 45 mit dem Anschluss 30 und einen gegenüberliegenden zweiten Teil 46 umfasst. In der Schalung ist eine weiche Einlage 47, beispielsweise aus Silikon, angeordnet, welche den Anlagebereich 31 aufweist und bei Benutzung am Penis anliegt. Die Teile 45, 46 der Schalung sind im unbelasteten Zustand des Applikators 111 voneinander beabstandet und können mit der Hand aufeinander zu bewegt werden. Dadurch wird die weiche Einlage 47 zusammengedrückt, so dass der Anpressdruck der Dichteinrichtung 32 auf den Penis vergrößert wird. Das Zusammendrücken der Einlage 47 kann durch Vorsehen einer oder mehrerer Einkerbungen 48 erleichtert werden. Durch Aufbringen eines ausreichenden Anpressdrucks wird der Druckraum 2 gegen die Umgebung abgedichtet und ein Druckwechselfeld kann auf den Penis appliziert werden.

Die Dichteinrichtung 32 ist ferner derart konzipiert, beispielsweise weich und abgerundet ausgebildet, dass der Applikator 111 auch entlang des Penisschafts bewegt werden kann. Es ist möglich, dass auch bei Bewegung ein ausreichender Anpressdruck und damit eine ausreichende Abdichtung des Druckraums 2 zum Applizieren eines Druckwechselfeldes erreicht wird. Wenn der Anpressdruck jedoch zu gering ist, kann der Aufbau des Druckwechselfeldes jedoch beeinträchtigt sein. Bei Benutzung kann somit eine kombinierte Stimulation erreicht werden, wobei der Applikator 111 beispielsweise derart bewegt werden kann, dass er neben der erzeugten Reibung zumindest phasenweise in einer dichtenden Position auf dem Penis aufliegt, sodass in diesen Positionen eine pneumatische Stimulation mittels des Druckfeldes erzeugt wird. Die dichtende Position kann dabei abhängig von der Stelle des Penis auch in Bewegung sein. Beispielsweise kann eine bewegte Abdichtung im Bereich des Penisschafts einfacher sein als im Bereich der Eichel. Möglicherweise kann es von einem Benutzer jedoch gewünscht sein, die Druckfelderzeugungseinrichtung 1 abzuschalten und den Applikator nur zur manuellen Stimulation zu verwenden. Es können gegebenenfalls Strukturen, wie Noppen, Rippen und der gleichen (nicht dargestellt) auf der Oberfläche der Einlage 47, also im Anlagebereich 31, vorgesehen sein, um in diesem Fall die Stimulation durch Reibung zu verstärken.

In FIG. 17 ist der Applikator 111 verbunden mit einer Druckfelderzeugungseinrichtung 1 dargestellt. Es ist die Hand eines Benutzers angedeutet, welche den Applikator 111 hält oder gegebenenfalls zusammendrückt. Hier ist der pneumatische Ausgang 35 der Druckfelderzeugungseinrichtung 1 direkt mit dem Anschluss 30 des Applikators 111 verbunden. Es versteht sich, dass zur Verbindung ein Verbindungsteil 8, beispielsweise ein Schlauch, wie oben beschrieben zwischen der Druckfelderzeugungseinrichtung 1 und dem Applikator 111 angeordnet werden kann.

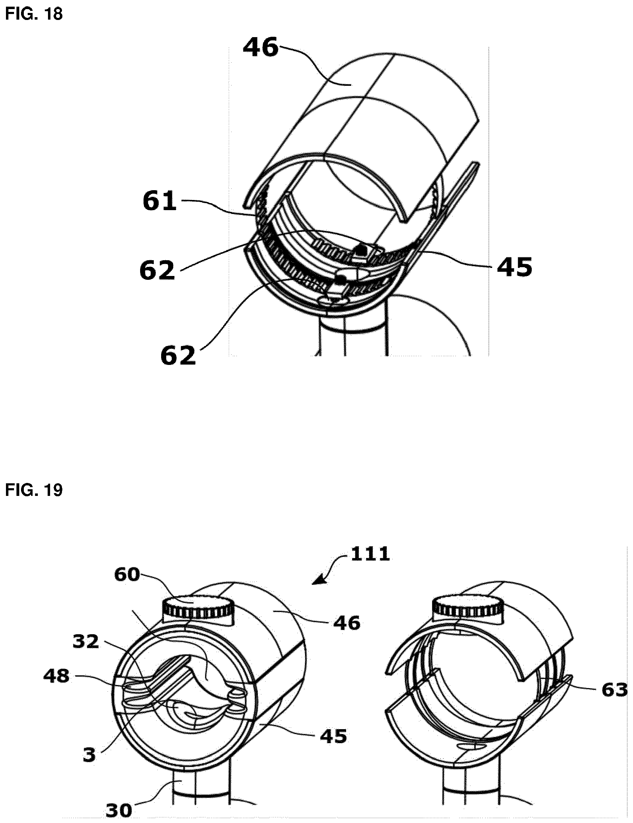

FIG. 18 zeigt einen Teil eines Ausführungsbeispiel eines Applikators 111, welches dem in FIG. 16 gezeigten ähnlich ist, wird eine Erhöhung der Anpresskraft ebenso durch manuelles Zusammendrücken der beiden Teile 45, 46 der Schalung erreicht. Es ist jedoch eine Fixiereinrichtung vorgesehen, welche die Schalung in einer gewünschten Stellung fixiert und den Anpressdruck auch nach Loslassen des Applikators aufrechterhält. Insbesondere ist ein Rastmechanismus 61 vorgesehen, wodurch die Spannkraft, die durch das Zusammendrücken entsteht, aufrecht erhalten bleibt auch wenn keine Kraft von außen auf die Schalung wirkt. Dies wird durch Ineinandergreifen entsprechender Oberflächenprofile, wie beispielsweise Zähnen, erreicht. Ein Mechanismus 62 ist vorgesehen, welcher durch geeignetes Verschieben der Oberflächenprofile relativ zueinander den Rastmechanismus 61 löst.

In dem in FIG. 19 dargestellten Ausführungsbeispiel eines Applikators 111, welches dem in FIG. 16 gezeigten ansonsten ähnlich ist, wird eine Erhöhung der Anpresskraft mittels einer Spanneinrichtung erreicht. Durch Betätigen der Spanneinrichtung, beispielsweise mittels eines Drehknopfes 60 werden Zugseile 63 gespannt. Die Zugseile 63 können sich in einem Führungskanal (nicht dargestellt) in den beiden Teilen 45 und 46 der Schalung befinden. Eine Drehbewegung des Drehknopfes 60 spannt die Zugseile 63, wodurch die Teile 45 und 46 aufeinander zu bewegt werden und die Anpresskraft auf den Penis erhöht wird. Die Spanneinrichtung besitzt vorzugsweise eine Fixiereinrichtung (nicht dargestellt) mit einer Einrastfunktion, die verhindert, dass die aufgebrachte Spannkraft ungewollt gelöst wird. Es ist vorteilhaft ein Mechanismus zum Lösen der Einrastfunktion vorgesehen.

In dem in FIG. 20 dargestellten Ausführungsbeispiel eines Applikators 111, welches dem in FIG. 16 gezeigten ansonsten ähnlich ist, wird eine Erhöhung der Anpresskraft mittels zumindest eines Luftkissens 64 erreicht, welches zwischen der hier einteilig ausgebildeten Schalung 49 und der Einlage 47 angeordnet ist. Durch Aufpumpen des Luftkissens 64, insbesondere mit Luft, kann der Durchmesser des Aufnahmeraums 34 des Applikators 111 zumindest abschnittsweise reduziert und der Anpressdruck auf den Penis erhöht werden. Ein Einwegventil (nicht dargestellt) kann vorgesehen sein, um die Luft aus dem Luftkissen 64 zu entlassen und so den Anpressdruck wieder zu reduzieren.

Gemäß dem in FIG. 21 gezeigten Ausführungsbeispiel weist der Applikator 211 eine flügelähnliche Schalung mit einem ersten Teil 245 und einem zweiten Teil 246 auf, die über ein Scharnier 250 beweglich, insbesondere verschwenkbar miteinander verbunden sind. Der Anlagebereich 31 umschließt den Penis nicht vollständig. Es ist vorzugsweise eine Torsionsfeder (nicht dargestellt) vorgesehen, welche eine Bewegung der Teile 245 und 246 der Schalung voneinander weg in eine geöffnete Position erzwingt. Eine Fixiereinrichtung (nicht dargestellt) mit einer Einrastfunktion kann vorgesehen sein, welche den Applikator 211 in einer von einem Benutzer durch Aufbringen einer Kraft bewirkte verengten Stellung fixiert. Ein Mechanismus zum Lösen der eingerasteten Stellung kann ebenso vorgesehen sein.

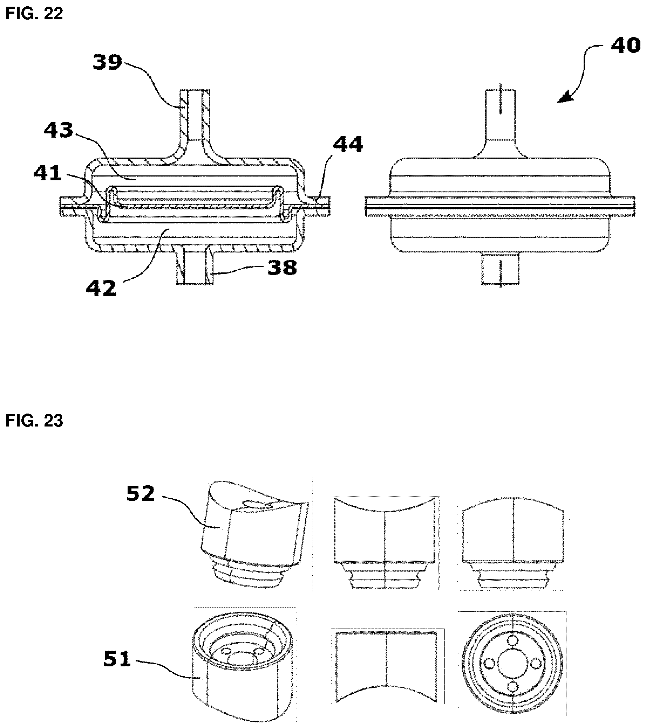

FIG. 22 zeigt eine Koppelungseinrichtung 40, welche insbesondere als Rückflussverschluss dient. Aufgrund hygienischer Gesichtspunkte ist bei der Verwendung eines Verbindungsteils 8 in Form eines Schlauches darauf zu achten, dass kein Sekret, infolge von Kapillarwirkung, in den Schlauch gesogen wird und sich darin auch nicht ablagert. Die Koppelungseinrichtung 40 wird zwischen dem Schlauch 8 und dem Applikator 11 oder zwischen zwei Abschnitten 38, 39 des Schlauchs 8 angebracht, wobei der Abschnitt 38 zur Druckfelderzeugungseinrichtung 1 führt, während der Abschnitt 39 zum Applikator 11 führt. Vorteilhaft ist die Koppelungseinrichtung 40 nahe am Applikator 11 positioniert, sodass der zu reinigende Abschnitt 39 des Schlauchs 8, welcher zum Applikator 11 hin weist, möglichst kurz ist.

Die Koppelungseinrichtung 40 weist eine Membran 41 auf, welche einen Hohlraum der Koppelungseinrichtung 40 in zwei Kammern 42, 43 teilt, sodass kein Fluid von der Kammer 43 in die Kammer 42 gelangen kann, also in Richtung vom Applikator 11 zur Druckfelderzeugungseinrichtung 1. Eine Druckänderung, insbesondere aufgrund des von der Druckfelderzeugungseinrichtung 1 erzeugten Druckwechselfeldes bewirkt eine Auslenkung der Membran 41, so dass das Druckwechselfeld trotz Verhinderung eines Fluidflusses übertragen wird. Insbesondere zur Reinigung kann die Koppelungseinrichtung 40 durch Lösen eines Befestigungsmechanismus 44 getrennt und der Abschnitt 39 des Schlauchs 8 mit dem Applikator 11 gereinigt werden. Eine Reinigung des Abschnitts 38 des Schlauchs 8 ist nicht notwendig, da aufgrund der fluidundurchlässigen Membran 41 kein Fluid in den Abschnitt 38 gelangen kann.

In FIG. 23 ist beispielhaft ein Ausführungsbeispiel eines Verbindungsmechanismus in Form einer fluiddichten Steckverbindung dargestellt. Mittels des Verbindungsmechanismus kann ein beliebiger Applikator 11, beispielweise einer der oben beschriebenen Applikatoren, direkt mit einer Druckfelderzeugungseinrichtung 1 verbunden werden. Alternativ kann der Verbindungsmechanismus zum Verbinden eines Verbindungsteils 8, beispielsweise eines Schlauchs, mit einem Applikator 11 oder einer Druckfelderzeugungseinrichtung 1 oder beiden verwendet werden. Eine Steckverbindung erlaubt ein einfaches Zusammensetzen einer Stimulationsvorrichtung. Die in FIG. 23 gezeigte Steckverbindung weist einen ersten Teil 51 mit einer Aufnahme auf, in welche ein Fortsatz eines zweiten Teils 52 eingeführt werden kann. Zur Abdichtung kann beispielsweise ein O-Ring in zumindest einem der Steckerteile oder in beiden vorgesehen sein.

Es versteht sich, dass beliebige Aspekte der oben beschriebenen bevorzugten Ausführungsbeispiele auf geeignete Weise miteinander kombiniert werden können. Insbesondere sind die bevorzugten Ausführungsbeispiele lediglich beispielhaft. Beispielsweise können verschiedene Aspekte des Applikators, wie beispielsweise Dichteinrichtung, Druckraum, Öffnung, Anlagebereich, Aufnahmeraum und Halteeinrichtung auf beliebige Weise kombiniert werden, um einen erfindungsgemäßen Applikator zum Applizieren eines Druckwechselfelds auf einen zu stimulierenden Bereich eines Penis, insbesondere die Eichel, vorzugsweise das Frenulum, zu schaffen. Auch kann unabhängig von der konkreten Ausgestaltung des Applikators, der Applikator direkt mit einer Druckfelderzeugungseinrichtung verbunden oder verbindbar sein, oder dazu ein Verbindungsteil, wie ein Schlauch bereitgestellt sein. Ferner kann vorgesehen sein, dass einer oder mehrere der beschriebenen Applikatoren oder auch mehrere Applikatoren des gleichen Typs in einem Set zusammen mit einer Druckfelderzeugungseinrichtung und gegebenenfalls einem geeigneten Verbindungsteil bereitgestellt werden.

## Patentansprüche

1. Applikator (11; 111; 211) für eine Stimulationsvorrichtung für einen männlichen Penis, umfassend:
- einen Applikatorkörper (29) mit einem Anlagebereich (31), welcher eingerichtet ist, beim Anlegen an einen Penis hieran wenigstens teilweise zur Anlage zu kommen;
- einen Druckraum (2), welcher an dem Applikatorkörper (29) gebildet und eingerichtet ist, von einer Druckfelderzeugungseinrichtung (1) ein pneumatisches Druckwechselfeld zu empfangen, wobei der Druckraum (2) eine Öffnung (3) im Anlagebereich (31) aufweist, so dass das Druckwechselfeld über die Öffnung (3) auf einen zu stimulierenden Bereich des Penis applizierbar ist; und
- eine Dichteinrichtung (32), welche an dem Applikatorkörper (29) gebildet und eingerichtet ist, beim Anlegen des Applikatorkörpers (29) an den Penis den Druckraum (2) gegenüber der Umgebung abzudichten;
wobei der Anlagebereich (31) des Applikatorkörpers (29) einen Aufnahmeraum (34) definiert, der an den Anlagebereich (31) grenzt,
wobei die Öffnung (3) des Druckraums (2) direkt in den Aufnahmeraum mündet, wobei der Aufnahmeraum (34) zwei entlang einer Längsrichtung gegenüberliegende offene Enden aufweist,
**dadurch gekennzeichnet,**
**dass** der Aufnahmeraum (34) eingerichtet ist, den Penis zumindest teilweise aufzunehmen, so dass der Anlagebereich (31) entlang zumindest eines Teils des Umfangs des Penis zur Anlage kommt, und
**dass** der Penis durch eines der Enden in den Applikator (11; 111; 211) einführbar ist.

2. Applikator nach Anspruch 1, wobei die Dichteinrichtung (32) im Anlagebereich (31) gebildet und eingerichtet ist, beim Anlegen des Applikatorkörpers (29) zumindest abschnittsweise am Penis zur Anlage zu kommen, um den Druckraum (2) gegenüber der Umgebung abzudichten.

3. Applikator nach Anspruch 1 oder 2, wobei die Dichteinrichtung (32) um die Öffnung des Druckraums herum umlaufend gebildet ist.

4. Applikator nach mindestens einem der vorhergehenden Ansprüche, wobei im Anlagebereich (31) des Applikatorkörpers (29) zumindest ein anatomisch geformter Vorsprung (33) angeordnet ist, welcher vorzugsweise zumindest abschnittsweise einem Verlauf einer Eichelkranzfurche (25) des Penis folgt.

5. Applikator nach mindestens einem der vorhergehenden Ansprüche, wobei der Druckraum (2) eingerichtet ist, beim Anlegen des Applikatorkörpers (29) an den Penis ein Frenulum (24) des Penis zumindest teilweise aufzunehmen.

6. Applikator nach mindestens einem der vorhergehenden Ansprüche, des Weiteren umfassend einen Anschluss (30), welcher an dem Applikatorkörper (29) angeordnet und eingerichtet ist, an eine Druckfelderzeugungseinrichtung (1) angeschlossen zu werden und von dieser ein pneumatisches Druckwechselfeld zu empfangen, wobei der Druckraum (2) mit dem Anschluss (30) fluidisch verbunden ist.

7. Applikator nach Anspruch 6, wobei im Applikatorkörper (29) ein Fluidkanal (7) gebildet ist, über welchen der Anschluss (30) und der Druckraum (2) fluidisch verbunden sind.

8. Applikator nach mindestens einem der vorhergehenden Ansprüche, wobei der Aufnahmeraum (34) entlang seines Umfangs geschlossen ist.

9. Applikator nach mindestens einem der vorhergehenden Ansprüche, des Weiteren umfassend eine Halteeinrichtung (26), welche eingerichtet ist, den Applikatorkörper (29) nach einem Anlegen am Penis zu halten, wobei die Halteeinrichtung (26) vorzugsweise weiter eingerichtet ist, einen Anpressdruck der Dichteinrichtung (32) an den Penis zu erhöhen.

10. Applikator nach Anspruch 9, wobei sich die Halteeinrichtung (26) von dem Applikatorkörper (29) erstreckt und eingerichtet ist, an zumindest einem Teil des Umfangs des Penis zur Anlage zu kommen.

11. Applikator nach Anspruch 9 oder 10, wobei die Halteeinrichtung (26) eine Fixiereinrichtung (27) aufweist, welche eingerichtet ist, die Halteeinrichtung (27) in einer gewünschten Stellung zu fixieren.

12. Stimulationsvorrichtung für einen männlichen Penis, umfassend
- einen Applikator (11; 111; 211) nach mindestens einem der vorhergehenden Ansprüche und
- eine Druckfelderzeugungseinrichtung (1), welche eingerichtet ist, ein pneumatisches Druckwechselfeld zu erzeugen und welche einen pneumatischen Ausgang (35) aufweist, über den ein erzeugtes pneumatisches Druckwechselfeld ausgebbar ist, wobei der pneumatische Ausgang (35) mit dem Applikator (11) verbindbar oder verbunden ist, sodass das pneumatische Druckwechselfeld von der Druckfelderzeugungseinrichtung (1) auf den Druckraum (2) des Applikators (11) übertragbar ist.

13. Stimulationsvorrichtung nach Anspruch 12, wobei der Applikator (11; 111; 211) einen Anschluss (30) umfasst, welcher mit dem Druckraum (2) fluidisch verbunden und mit dem pneumatischen Ausgang (35) verbindbar oder verbunden ist, sodass das pneumatische Druckwechselfeld von der Druckfelderzeugungseinrichtung (1) auf den Druckraum (2) des Applikators (11) übertragbar ist,
wobei die Stimulationsvorrichtung des Weiteren ein Verbindungsteil (8) mit einem ersten Anschluss (36), welcher eingerichtet ist, mit dem pneumatischen Ausgang (35) der Druckfelderzeugungseinrichtung (1) fluidisch verbunden zu werden, und einem zweiten Anschluss (37), welcher eingerichtet ist, mit dem Anschluss (30) des Applikators (11) fluidisch verbunden zu werden, so dass das pneumatische Druckwechselfeld mittels des Verbindungsteils (8) von der Druckfelderzeugungseinrichtung (1) auf den Druckraum (2) des Applikators (11) übertragbar ist, umfasst.

14. Stimulationsvorrichtung nach Anspruch 13, wobei das Verbindungsteil (8) eine Koppelungseinrichtung (40) aufweist, welche zwischen dem ersten Anschluss (36) und dem zweiten Anschluss (37) des Verbindungsteils (8) angeordnet und eingerichtet ist, das pneumatische Druckwechselfeld zu übertragen und einen Fluidfluss zu verhindern, wobei die Koppelungseinrichtung (40) vorzugsweise eine flexible und fluidundurchlässige Membran (41) aufweist.

15. Artikel mit einer Stimulationsvorrichtung für einen männlichen Penis nach mindestens einem der Ansprüche 12 bis 14, wobei der Applikator (11; 111; 211) ein erster Applikator ist, und wenigstens einem weiteren Applikator (11; 111; 211) nach mindestens einem der Ansprüche 1 bis 11, welcher von dem ersten Applikator verschieden ist.

## Claims

1. An applicator (11; 111; 211) for a stimulation device for a male penis, comprising:
- an applicator body (29) having a contact region (31) configured to come at least partially into contact therewith when applied to a penis;
- a pressure chamber (2) formed on the applicator body (29) and configured to receive a pneumatic alternating pressure field from a pressure field generating device (1), wherein the pressure chamber (2) has an opening (3) in the contact region (31) such that the alternating pressure field can be applied via the opening (3) to a region of the penis to be stimulated; and
- a sealing device (32) formed on the applicator body (29) and configured to seal the pressure chamber (2) from the surroundings when the applicator body (29) is applied to the penis;
wherein the contact region (31) of the applicator body (29) defines a receiving space (34) adjoining the contact region (31), wherein the opening (3) of the pressure chamber (2) opens directly into the receiving space, wherein the receiving space (34) has two open ends opposite one another along a longitudinal direction,
**characterized in that** the receiving space is configured to at least partially receive the penis such that the contact region (31) comes into contact along at least part of the circumference of the penis, and **in that** the penis can be introduced into the applicator (11; 111; 211) through one of the ends.

2. The applicator according to claim 1, wherein the sealing device (32) is formed in the contact region (31) and configured to come at least partially into contact with the penis when the applicator body (29) is applied in order to seal the pressure chamber (2) from the surroundings.

3. The applicator according to claim 1 or 2, wherein the sealing device (32) is formed peripherally around the opening of the pressure chamber.

4. The applicator according to at least one of the preceding claims, wherein at least one anatomically shaped projection (33) is arranged in the contact region (31) of the applicator body (29), which preferably at least partially follows a course of a glanical ridge (25) of the penis.

5. The applicator according to at least one of the preceding claims, wherein the pressure chamber (2) is configured to at least partially receive a frenulum (24) of the penis when the applicator body (29) is applied to the penis.

6. The applicator according to at least one of the preceding claims, further comprising a connector (30) arranged on the applicator body (29) and configured to be connected to a pressure field generating device (1) and to receive a pneumatic alternating pressure field therefrom, wherein the pressure chamber (2) is fluidically connected to the connector (30).

7. The applicator according to claim 6, wherein a fluid channel (7) is formed in the applicator body (29), via which the connector (30) and the pressure chamber (2) are fluidically connected.

8. The applicator according to at least one of the preceding claims, wherein the receiving space (34) is closed along its circumference.

9. The applicator according to at least one of the preceding claims, further comprising a holding device (26) configured to hold the applicator body (29) after application to the penis, wherein the holding device (26) is preferably further configured to increase a contact pressure of the sealing device (32) on the penis.

10. The applicator according to claim 9, wherein the holding device (26) extends from the applicator body (29) and is configured to come into contact with at least part of the circumference of the penis.

11. The applicator according to claim 9 or 10, wherein the holding device (26) has a fixing device (27) configured to fix the holding device (27) in a desired position.

12. A stimulation device for a male penis, comprising
- an applicator (11; 111; 211) according to at least one of the preceding claims and
- a pressure field generating device (1) configured to generate a pneumatic alternating pressure field and having a pneumatic output (35) via which a generated pneumatic alternating pressure field can be output, wherein the pneumatic output (35) is connectable or connected to the applicator (11) such that the pneumatic alternating pressure field can be transferred from the pressure field generating device (1) to the pressure chamber (2) of the applicator (11).

13. The stimulation device according to claim 12, wherein the applicator (11; 111; 211) comprises a connector (30) fluidically connected to the pressure chamber (2) and connectable or connected to the pneumatic output (35) such that the pneumatic alternating pressure field can be transferred from the pressure field generating device (1) to the pressure chamber (2) of the applicator (11),
wherein the stimulation device further comprises a connecting part (8) having a first connector (36) configured to be fluidically connected to the pneumatic output (35) of the pressure field generating device (1) and a second connector (37) configured to be fluidically connected to the connector (30) of the applicator (11) such that the pneumatic alternating pressure field can be transferred from the pressure field generating device (1) to the pressure chamber (2) of the applicator (11) by means of the connecting part (8).

14. The stimulation device according to claim 13, wherein the connecting part (8) has a coupling device (40) arranged between the first connector (36) and the second connector (37) of the connecting part (8) and configured to transfer the pneumatic alternating pressure field and to prevent a fluid flow, wherein the coupling device (40) preferably has a flexible and fluid-impermeable membrane (41).

15. An article comprising a stimulation device for a male penis according to at least one of claims 12 to 14, wherein the applicator (11; 111; 211) is a first applicator, and at least one further applicator (11; 111; 211) according to at least one of claims 1 to 11, which is different from the first applicator.

## Revendications

1. Applicateur (11 ; 111 ; 211) pour un dispositif de stimulation pour un pénis mâle, comprenant :
- un corps d'applicateur (29) avec une zone d'appui (31), qui est conçue pour venir en appui au moins partiellement contre celui-ci lors de l'application contre un pénis ;
- une chambre de pression (2), qui est formée sur le corps d'applicateur (29) et conçue pour recevoir un champ de pression alternatif pneumatique d'un dispositif de génération de champ de pression (1), la chambre de pression (2) présentant une ouverture (3) dans la zone d'appui (31), de sorte que le champ de pression alternatif peut être appliqué par le biais de l'ouverture (3) sur une zone à stimuler du pénis ; et
- un dispositif d'étanchéité (32), qui est formé sur le corps d'applicateur (29) et conçu pour étanchéifier la chambre de pression (2) par rapport à l'environnement lors de l'application du corps d'applicateur (29) contre le pénis ;
la zone d'appui (31) du corps d'applicateur (29) définissant un espace de réception (34), qui est adjacent à la zone d'appui (31), l'ouverture (3) de la chambre de pression (2) débouchant directement dans l'espace de réception, l'espace de réception (34) présentant deux extrémités ouvertes opposées le long d'une direction longitudinale, **caractérisé en ce que** l'espace de réception est conçu pour recevoir au moins partiellement le pénis, de sorte que la zone d'appui (31) vient en appui le long d'au moins une partie de la périphérie du pénis, et **en ce que** le pénis peut être introduit par l'une des extrémités dans l'applicateur (11 ; 111 ; 211).

2. Applicateur selon la revendication 1, le dispositif d'étanchéité (32) étant formé dans la zone d'appui (31) et conçu pour venir en appui au moins en partie contre le pénis lors de l'application du corps d'applicateur (29), afin d'étanchéifier la chambre de pression (2) par rapport à l'environnement.

3. Applicateur selon la revendication 1 ou 2, le dispositif d'étanchéité (32) étant formé de manière périphérique autour de l'ouverture de la chambre de pression.

4. Applicateur selon au moins l'une quelconque des revendications précédentes, au moins une saillie (33) de forme anatomique étant disposée dans la zone d'appui (31) du corps d'applicateur (29), laquelle suit de préférence au moins en partie un tracé d'un sillon de couronne (25) du pénis.

5. Applicateur selon au moins l'une quelconque des revendications précédentes, la chambre de pression (2) étant conçue pour recevoir au moins partiellement un frenulum (24) du pénis lors de l'application du corps d'applicateur (29) contre le pénis.

6. Applicateur selon au moins l'une quelconque des revendications précédentes, comprenant en outre un raccord (30), qui est disposé sur le corps d'applicateur (29) et conçu pour être raccordé à un dispositif de génération de champ de pression (1) et pour recevoir de celui-ci un champ de pression alternatif pneumatique, la chambre de pression (2) étant connectée fluidiquement au raccord (30).

7. Applicateur selon la revendication 6, un canal de fluide (7) étant formé dans le corps d'applicateur (29), par le biais duquel le raccord (30) et la chambre de pression (2) sont connectés fluidiquement.

8. Applicateur selon au moins l'une quelconque des revendications précédentes, l'espace de réception (34) étant fermé le long de sa périphérie.

9. Applicateur selon au moins l'une quelconque des revendications précédentes, comprenant en outre un dispositif de retenue (26), qui est conçu pour retenir le corps d'applicateur (29) après une application contre le pénis, le dispositif de retenue (26) étant de préférence en outre conçu pour augmenter une pression d'appui du dispositif d'étanchéité (32) contre le pénis.

10. Applicateur selon la revendication 9, le dispositif de retenue (26) s'étendant depuis le corps d'applicateur (29) et étant conçu pour venir en appui contre au moins une partie de la périphérie du pénis.

11. Applicateur selon la revendication 9 ou 10, le dispositif de retenue (26) présentant un dispositif de fixation (27), qui est conçu pour fixer le dispositif de retenue (27) dans une position souhaitée.

12. Dispositif de stimulation pour un pénis mâle, comprenant
- un applicateur (11 ; 111 ; 211) selon au moins l'une quelconque des revendications précédentes et
- un dispositif de génération de champ de pression (1), qui est conçu pour générer un champ de pression alternatif pneumatique et qui présente une sortie pneumatique (35), par le biais de laquelle un champ de pression alternatif pneumatique généré peut être délivré, la sortie pneumatique (35) pouvant être connectée ou étant connectée à l'applicateur (11), de sorte que le champ de pression alternatif pneumatique peut être transmis du dispositif de génération de champ de pression (1) à la chambre de pression (2) de l'applicateur (11).

13. Dispositif de stimulation selon la revendication 12, l'applicateur (11 ; 111 ; 211) comprenant un raccord (30), qui est connecté fluidiquement à la chambre de pression (2) et qui peut être connecté ou est connecté à la sortie pneumatique (35), de sorte que le champ de pression alternatif pneumatique peut être transmis du dispositif de génération de champ de pression (1) à la chambre de pression (2) de l'applicateur (11),
le dispositif de stimulation comprenant en outre une partie de connexion (8) avec un premier raccord (36), qui est conçu pour être connecté fluidiquement à la sortie pneumatique (35) du dispositif de génération de champ de pression (1), et un deuxième raccord (37), qui est conçu pour être connecté fluidiquement au raccord (30) de l'applicateur (11), de sorte que le champ de pression alternatif pneumatique peut être transmis au moyen de la partie de connexion (8) du dispositif de génération de champ de pression (1) à la chambre de pression (2) de l'applicateur (11).

14. Dispositif de stimulation selon la revendication 13, la partie de connexion (8) présentant un dispositif de couplage (40), qui est disposé entre le premier raccord (36) et le deuxième raccord (37) de la partie de connexion (8) et qui est conçu pour transmettre le champ de pression alternatif pneumatique et pour empêcher un écoulement de fluide, le dispositif de couplage (40) présentant de préférence une membrane flexible et imperméable aux fluides (41).

15. Article comprenant un dispositif de stimulation pour un pénis mâle selon au moins l'une quelconque des revendications 12 à 14, l'applicateur (11 ; 111 ; 211) étant un premier applicateur, et au moins un autre applicateur (11 ; 111 ; 211) selon au moins l'une quelconque des revendications 1 à 11, qui est différent du premier applicateur.
